(19) Europäisches Patentamt
European Patent Office
Office européen des brevets

(11) **EP 4 328 880 A2**

(12) **EUROPÄISCHE PATENTANMELDUNG**

(43) Veröffentlichungstag:
**28.02.2024 Patentblatt 2024/09**

(51) Internationale Patentklassifikation (IPC):
**G07D 7/206** (2016.01)

(21) Anmeldenummer: **23000176.0**

(22) Anmeldetag: **04.06.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.06.2019 US 201962856795 P**

(62) Dokumentnummer(n) der früheren Anmeldung(en) nach Art. 76 EPÜ:
**20732154.8 / 3 981 016**

(71) Anmelder:
• **Brücher, Christoph**
**65760 Eschborn (DE)**

• **Windhab, Norbert**
**65719 Hofheim (DE)**

(72) Erfinder: **Die Erfindernennung liegt noch nicht vor**

(74) Vertreter: **Ackermann, Joachim**
**Antoniterstraße 9**
**65929 Frankfurt am Main (DE)**

Bemerkungen:
Diese Anmeldung ist am 07.12.2023 als Teilanmeldung zu der unter INID-Code 62 erwähnten Anmeldung eingereicht worden.

(54) **EINDEUTIGE IDENTIFIZIERUNG UND AUTHENTIFIZIERUNG VON PRODUKTEN**

(57) Beschrieben werden Verfahren zur eindeutigen Identifizierung und Authentifizierung von Produkten zum Zwecke der besseren Erkennung von Produktfälschungen und Bekämpfung von Produktpiraterie sowie die damit identifizierten Produkte.

Eines der Verfahren umfasst den Verfahrensschritt des Aufbringens an/auf/in das Produkt einer paramagnetische Phasen enthaltenden Identifizierungs-Stoffzumischung oder Identifizieren eines Produktes, welches eine paramagnetische Phasen enthaltende Identifizierungs-Stoffzumischung enthält, die ein ESR-Fingerprint-Spektrum aufweist, das eine eindeutige Identifizierung des Produktes erlaubt, und Erzeugung und Speicherung einer digitalen Repräsentation des ESR-Fingerprint-Spektrums fälschungs- und manipulationssicher auf einem Blockchain-Netzwerk. Ein derartiges ESR-Fingerprint-Spektrum ist mit einfachen Mitteln messbar, jedoch nur schwer zu kopieren oder zu fälschen. Das Verfahren betrifft einen individualisierbaren "Femto-Tag" für Femto-Ledger und bildet eine wichtige Schnittstelle für sogenannte "Internet of Things (IOT) Anwendungen".

Fig. 1

S1: Aufbringen eine paramagnetische Phasen enthaltenden Identifizierungs-Stoffzumischung

S2: Aufnahme eines ESR-Fingerprint-Spektrums des Produktes

S3: Erzeugung und Speicherung einer digitalen Repräsentation des ESR-Fingerprint-Spektrums

S4: Messung des ESR-Spektrums eines zu authentifizierenden Produktes

S5: Erzeugung einer digitalen Repräsentation des gemessenen ESR-Spektrums

S6: Verifizierung des Produktes durch Vergleich der digitalen Repräsentation des gemessenen ESR-Spektrums des zu authentifizierenden Produktes mit digitalen Repräsentationen gespeicherter ESR-Fingerprint-Spektren

EP 4 328 880 A2

## Beschreibung

### Gebiet der Erfindung

Die Erfindung betrifft Verfahren zur eindeutigen Identifizierung und Authentifizierung von Produkten sowie eindeutig identifizierbare und authentifizierbare Produkte. Das Verfahren stellt somit einen individualisierbaren "Femto-Tag" für Femto-Ledger bereit und bildet eine wichtige Schnittstelle für sogenannte "Internet of Things (IOT) Anwendungen".

### Hintergrund der Erfindung

Durch Fälschungen und Raubkopien hochwertiger Produkte entstehen jedes Jahr enorme wirtschaftliche Schäden. Im Falle von Medikamenten, Lebensmitteln sowie Zuliefer- und Ersatzteilen in sicherheitskritischen Branchen wie der Luftfahrt und der Automobilindustrie können Fälschungen außerdem ein hohes Risiko für Leib und Leben vieler Menschen darstellen.

Aufgrund der Herstellung von authentischen Kopien eines Produktes ist es häufig schwierig, wenn nicht sogar unmöglich, Originalprodukte und Plagiate voneinander zu unterscheiden. Insbesondere bei Kunststoffen oder Beschichtungen ist es nicht möglich oder sinnvoll, eine Seriennummer oder andere, eine Produktidentifizierung erlaubende Information in das Material des Produktes selbst einzubringen.

Nach dem Stand der Technik werden zum Schutz vor Kopien oder Fälschungen neben den bekannten Sicherheitsetiketten in Form von Schildersätzen zum Beispiel im Automobilbereich Etiketten eingesetzt, die an den Produkten angebracht werden und verschiedene offene und verdeckte Sicherheitsmerkmale aufweisen, die beispielsweise mit einem hoch auflösenden Laser in das Informationsfeld eingeschrieben werden (DE 20 2007 017 753 U1). Ferner existieren Hologramme (DE 10 030 629 A1), Lithogramme mit Datenträgerfeldern, Barcodes und Matrixcodes, die an Produkten angebracht werden und die herkunftsspezifische Informationen direkt am Produkt auf voneinander unabhängigen Informationsebenen darstellen können. Der Nachteil dieser bekannten Schutzmechanismen besteht darin, dass diese mit einem hohen technischen und kostenintensiven Aufwand hergestellt und an den Produkten - in der Regel gut sichtbar - angebracht werden müssen.

Aus der US 2006/054825 A1 ist ein Verfahren zur Identifizierung und Authentifizierung unterschiedlicher Objekte oder Substanzen bekannt, wobei dieses Verfahren ein Datenverarbeitungssystem benutzt, welches an Mittel zur Spektrophotometrie angekoppelt ist. Das Verfahren zeichnet sich insbesondere dadurch aus, dass es zwei Phasen umfasst: während einer Initialphase werden eine Vielzahl von chemischen Markern ausgewählt, dann eine Kombination von Markierungen in jedes der Objekte oder Substanzen zugeordnet und eingebracht, ein Authentifizierungs-Code erstellt, der Authentifizierungs-Code gespeichert und ein Identifizierungs-Code zu dem Objekt oder der Substanz zugeordnet und auch dieser Code gespeichert, wonach eine Zuordnung zwischen dem Identifizierungs-Code und dem Authentifizierungs-Code hergestellt wird. Es schließt sich eine Identifizierungs- und Authentifizierungs-Phase an, die eine theoretische Identifizierung des Objekts oder der Substanz durch Auslesen des mit dem Objekt oder der Substanz verknüpften Identifizierungs-Codes, die spektrophotometrische Analyse des Objekts oder der Substanz und die Ermittlung des Authentifizierungs-Codes des Objekts oder Substanz, die Authentifizierung des Objekts oder der Substanz in dem Falle, in dem der theoretische Identifizierungs-Code mit dem Authentifizierungs-Code übereinstimmt und schließlich die Abgabe eines Freigabesignals, in dem Falle, dass eine Übereinstimmung hergestellt worden ist, oder eines Alarmsignals in dem Falle, dass keine Übereinstimmung zwischen Authentifizierungs-Code und Identifizierungs-Code vorliegt, umfasst.

Aus der DE 44 45 004 A1 ist eine Zusammensetzung zur delokalisierten Kennzeichnung von Gegenständen, ihre Herstellung und Verwendung bekannt. Die Zusammensetzung ermöglicht eine Kennzeichnung von Gegenständen, die die Fälschung oder unrechtmäßige Verwendung oder Verwertung dieser Gegenstände erschwert. Sie ist dadurch gekennzeichnet, dass sie chemische Elemente mit einer $K\alpha$-Linie von 3,69 keV bis 76,315 keV in definierten Verteilungen enthält, wobei die physikalischen Eigenschaften dieser Substanz bzw. ihre elementare und/oder mengenmäßige Zusammensetzung als delokalisierte, mit dem bloßen Auge nicht erkennbare Information dient.

Die DE 10 2008 060 675 A1 beschreibt ein Verfahren zur eindeutigen Identifizierung und Authentifizierung eines Produkts zum Schutz vor Plagiaten mittels Verwendung von Markern, wobei ein pulverförmiger Marker im Werkstoff des zu schützenden Produkts eingebunden ist, welcher Marker einen inerten Träger und chemische Elemente in einem zuvor festgelegten Elementencode aus einer Anzahl an chemischen Elementen und einer zuvor definierten Verschlüsselungssequenz mit einer definierten Anordnung der chemischen Elemente und festgelegten relativen Konzentrationen der chemischen Elemente umfasst. Dabei sind der Träger und die chemischen Elemente untrennbar miteinander verbunden, wobei das Verfahren die Schritte umfasst: (i) Bestimmen der chemischen Elemente und deren Gehalte im markierten Werkstoff, (ii) Vergleichen der in Schritt (i) bestimmten Werte mit dem zuvor festgelegten Elementencode und der zuvor definierten Verschlüsselungssequenz.

Die US 2018/0335427 A1 beschreibt die Verwendung von Markern (Tags) zur Nachverfolgung und Identifizierung von pharmazeutischen und ernährungsphysiologischen Produkten, welche Marker mindestens ein paramagnetisches Mikroteilchen umfassen, wobei das mindestens eine paramagnetische Mikroteilchen eine nicht-kugelförmige Form, einen Formfaktor größer als eins, und mindestens eine einzigartige und nachweisbare Chemikalie umfasst, worin die mindestens eine einzigartige und nachweisbare Chemikalie mit dem mindestens einen paramagnetischen Mikropartikel verbunden ist. Die einzigartigen und nachweisbaren Chemikalien, die mit den paramagnetischen Mikropartikeln verbunden sind, werden etwa mittels Lichtabsorptionsspektroskopie, Ramanspektroskopie, Oberflächenplasmonresonanz, Fluoreszenz, elektrochemische Detektion, Ionenchromatographie und Enzym-Farbwechselchemie analysiert.

Nachteilig bei den vorstehend beschriebenen Verfahren ist unter anderem der hohe technischen Aufwand bei der Herstellung der Marker sowie der Verifizierung eines Produktes als authentisches Produkt.

Es besteht daher ein Bedarf an einfachen, preisgünstigen und wirkungsvollen Verfahren zur eindeutigen Identifizierung und Authentifizierung von Produkten, um Fälschungen oder unerlaubte Kopien zu erkennen.

## Zusammenfassung der Erfindung

Der vorliegenden Erfindung liegt die überraschende Erkenntnis zu Grunde, das sich Elektronenresonanz-Spektren (ESRSpektren) einerseits mit geringem Aufwand detektieren lassen und andererseits durch eine Kombination von paramagnetischen Phasen eine Vielzahl von eindeutigen ESR-Spektren, sogenannten ESR-Fingerprint-Spektren, gezielt erzeugen lassen und dem zu identifizierenden Produkt als Stoffzumischung beigefügt werden kann.

Die vorliegende Erfindung schlägt daher ein Produkt gemäß Anspruch 1 vor, aufweisend eine paramagnetische Phasen enthaltende Identifizierungs-Stoffzumischung, die ein ESR-Fingerprint-Spektrum aufweist, das eine eindeutige Identifizierung des Produktes erlaubt.

Die paramagnetischen Phasen der Identifizierungs-Stoffzumischung können dabei gebildet werden durch eine oder mehrere Phasen ausgewählt aus:

- paramagnetischen Zentren, bevorzugt S-Radikale, vorzugsweise ausgewählt aus Ultramarin,
- kollektiv ordnenden Zuständen ausgewählt aus ferro-, ferri-, und/oder antiferromagnetischen Zuständen, vorzugsweise ausgewählt aus Eisen-Sauerstoff-Verbindungen, besonders bevorzugt Magnetit oder einem Material mit Fe-O Phasen,
- einem Polymer, das ein paramagnetisches Zentrum, vorzugsweise isolierte Radikale aufweist,
- molekularen Paramagneten, und
- paramagnetischen Phasen mineralischer Stoffen und/oder Salze, insbesondere paramagnetische Phasen von $Al_2O_3$, $SiO_2$, natürlich oder künstlich dotiertem Diamant oder $ZrO_2$.

In einer Ausführungsform ist die Phase ausgewählt aus Kupfer(II)sulfat, Mangan(II)chlorid, Mangan(IV)Oxid, Zirconium(IV)oxid, Lactose-Monohydrat, Titandioxid, Homo- und Copolymeren, insbesondere (Meth)acrylatcopolymeren wie beispielsweise kommerziell erhältliches Eudragit® E, L, RL, FL 30 D der Evonik Industries AG oder Polylactid-co-Glycolid wie beispielsweise kommerziell erhältlich unter dem Handelsnamen Resomer® der Evonik Industries AG, natürlichem Ultramarinblau, D(-)-Mannit, Diamantpulver, Magnesiumoxid, Tintenschwarz, D(+)-Trehalose, mikrokristalliner Cellulose wie kommerziell erhältliches Avicel PH-101, Proteine, insbesondere fermentierte und/oder rekombinante, wie tripelhelikales Collagen, und Mischungen davon. In einer Ausführungsform werden diese Phasen, insbesondere vor Aufnahme des ESR-Fingerprint-Spektrums, durch Energieeintrag aktiviert, insbesondere durch Röntgenbestrahlung.

In einer Ausführungsform sind die Phasen ein Gemisch aus Lactose-Monohydrat, MCC, natürlichem Ultramarinblau, Diamantpulver, Kupfer(II)sulfat und Magnesiumoxid. In einer Ausführungsform sind die Phasen ein Gemisch aus natürlichem Ultramarinblau, Diamantpulver, Kupfer(II)sulfat und Magnesiumoxid, bevorzugt in äquivalenten Massen. In einer Ausführungsform wird ein Gemisch aus natürlichem Ultramarinblau, Diamantpulver, Kupfer(II)sulfat und Magnesiumoxid, in äquivalenten Massen mit Lactose-Monohydrat und MCC verdünnt, in einem Gewichtsverhältnis von 2/3 : 1/3 bis 1/8 : 7/8, wobei Lactose-Monohydrat und MCC ebenfalls in äquivalenten Massen zueinander eingesetzt werden. In einer Ausführungsform wird das Gemisch, insbesondere vor Aufnahme des ESR-Fingerprint-Spektrums, durch Energieeintrag aktiviert, insbesondere durch Röntgenbestrahlung.

In einer Ausführungsform sind die Phasen in 0,0005 bis 50 Gew.-%, bevorzugt 0,001 bis 20 Gew.-%, stärker bevorzugt in 0,01 bis 10 Gew.-% oder 0,01 bis 1 Gew.-%, basierend auf dem Gesamtgewicht der zu analysierenden Probe enthalten. In einer Ausführungsform sind die Phasen in 0,0005 bis 0,1 Gew.-%, basierend auf dem Gesamtgewicht der zu analysierenden Probe enthalten.

Die genannten Materialien haben dabei vorzugsweise nicht nur die gewünschten magnetischen Eigenschaften, sondern sind auch ungiftig und zum Verzehr geeignet, d.h. auch für den Einsatz in Arznei- und Lebensmitteln geeignet.

Die paramagnetischen Phasen der Identifizierungs-Stoffzumischung können hergestellt sein durch Verfahren umfassend Beschichtung, Beimischung, Dotierung, Sputtering, chemische Radikalerzeugung, Bestrahlung, insbesondere Röntgenstrahlung, und/oder durch Druckverfahren.

Die Druckverfahren können dabei umfassen Hochdruck, Tiefdruck, Durchdruck, Siebdruck, Flachdruck wie Offsetdruck, Digitaldruck, Stahlstich, Siebdruck, Elektrofotographie (Laserdruck), Pulverdruckverfahren, wie Xerox und Elektrospray, Elektrospinning, Fällungen, Papierschöpfung oder Schichtverfahren wie etwa Siebdruck, oder Direct Imaging. Geeignete Rohstoffe und Medien dafür sind beispielsweise Pasten, Tinten, Beizen, Gase und Dämpfe, Lacke, Matrizen, Pulver, Lösungen, Schmelzen, Gläser, und physikalisch aktive bzw. chemisch reaktive Formen davon.

Zur Dekodierung der im ESR-Spektrum codierten Information können dabei Resonanzfrequenzen, Linienform, Intensität, Signalkopplung und/oder räumliche Variation des ESR-Fingerprint-Spektrums auswertbar sein.

Vorzugsweise ist das ESR-Fingerprint-Spektrum der Identifizierungs-Stoffzumischung mechanisch und/oder thermisch stabil. Dadurch wird eine Produktidentifizierung auch nach einem mechanisch und/oder thermisch anspruchsvollen Transportweg gewährleistet. Alternativ kann beispielsweise jedoch ein temperaturempfindliches ESR-Spektrum zur Erfassung von unterbrochenen Kühlketten dienen, etwa bei Medikamenten oder Lebensmitteln.

Bei dem erfindungsgemäßen Produkt kann es sich beispielsweise um ein Pharmazeutikum oder Arzneimittel, ein Lebensmittel oder ein Vor- oder Zwischenprodukt oder regulierter Bestandteil davon handeln, etwa eine Verpackung, Blister, Behälter, wie Glas- und Polymerröhrchen, eine Spritze, Ampulle oder ein Reservoir für Flüssigkeiten.

Bei dem erfindungsgemäßen Produkt kann es sich auch um ein Gewebe, ein Textil- oder Lederprodukt, eine Münze, ein Geldschein, Wertpapier, Urkunde, Zertifikat oder eine Scheck- oder Chipkarte oder ein Teil davon wie Siegel oder Hüllen handeln, oder um ein einen Edel- oder Schmuckstein, ein Medizinprodukt, ein Implantat oder Transplantat, oder ein Ersatz oder Zulieferteil für ein Industrieprodukt.

Das ESR-Fingerprint-Spektrum kann das Produkt selbst, einen Hersteller, einen Herstellungsort, eine Herstellungszeit und/oder produktionsspezifische Daten wie etwa bestimmungsgemäßer Verwendungszweck, Rechte wie Lizenzen oder geographische Indikationen, autorisierende Institutionen wie Zulassungsbehörden oder dergleichen codieren.

Die Identifizierungs-Stoffzumischung und das zugehörige ESR-Spektrum kann für jedes individuelle Produkt oder für Produktchargen identisch gewählt werden.

Die Erfindung schlägt ferner ein Verfahren zur Herstellung eines eindeutig identifizierbaren Produktes vor, aufweisend den Verfahrensschritt des Auf- oder Einbringens an/auf/in das Produkt einer paramagnetische Phasen enthaltenden Identifizierungs-Stoffzumischung, die ein ESR-Fingerprint-Spektrum aufweist, das eine eindeutige Identifizierung des Produktes erlaubt, und Erzeugung und Speicherung einer digitalen Repräsentation des ESR-Fingerprint-Spektrums fälschungs- und manipulationssicher auf einem Blockchain-Netzwerk.

Die paramagnetischen Phasen der Identifizierungs-Stoffzumischung können dabei hergestellt werden durch Verfahren umfassend Beschichtung, Beimischung, Dotierung, Sputtering, chemische Radikalerzeugung und/oder Bestrahlung, insbesondere Röntgenstrahlung, Druck, Prägung, Schmelzen, Extrusion, Pressung, Granulierung, Spheronisierung, Sprühtrocknung, Additive Herstellung (3D-Druck), Thermotransfer, Heißprägen, Laserverfahren, Tintenstrahldruck und Holographiedruck.

Die Erfindung schlägt außerdem ein Verfahren zur Produktauthentifizierung vor, aufweisend die Verfahrensschritte:

(a) Auf- oder Einbringen eine paramagnetische Phasen enthaltenden Identifizierungs-Stoffzumischung an/auf/in ein Produkt oder Identifizieren eines Produktes, welches eine paramagnetische Phasen enthaltende Identifizierungs-Stoffzumischung enthält, welche Identifizierungs-Stoffzumischung ein ESR-Fingerprint-Spektrum aufweist, das eine eindeutige Identifizierung des Produktes erlaubt,

(b) Aufnahme eines ESR-Fingerprint-Spektrums des Produktes,

(c) Erzeugung und Speicherung einer digitalen Repräsentation des ESR-Fingerprint-Spektrums,

(d) Messung des ESR-Spektrums eines zu authentifizierenden Produktes und Erzeugung einer digitalen Repräsentation des gemessenen ESR-Spektrums,

(e) Verifizierung des zu authentifizierenden Produktes durch Vergleich der digitalen Repräsentation des gemessenen ESR-Spektrums des zu authentifizierenden Produktes mit digitalen Repräsentationen gespeicherter ESR-Fingerprint-Spektren.

Dabei kann die digitale Repräsentation eines ESR-Fingerprint-Spektrums einen aus dem ESR-Fingerprint-Spektrum abgeleiteten Hashwert enthalten. So können in dem ESR-Fingerprint-Spektrum Informationen vertrauliche Informationen codiert werden, da der Hashwert eine Ermittlung der codierten Ursprungsdaten mit vernünftigem Rechenaufwand nicht erlaubt, umgekehrt in Kenntnis der Ursprungsdaten deren Verifizierung leicht möglich ist (Einweg-Codierung).

Die einem Produkt zugeordneten digitalen Repräsentationen der ESR-Fingerprint-Spektren können fälschungs- und manipulationssicher auf einem Blockchain-Netzwerk abgespeichert werden. Dabei kann beispielsweise für jede abgespeicherte digitale Repräsentation eines ESR-Fingerprint-Spektrums ein eindeutiger non-fungible-token auf dem Blockchain-Netzwerk erzeugt werden, so dass jedem eindeutigen ESR-Fingerprint-Spektrum ein eindeutiger Token auf dem Blockchain-Netzwerk zugeordnet werden kann, wodurch beispielsweise Transaktionen (Verkäufe, Lizensierungen) betreffend die durch die ESR-Fingerprint-Spektren repräsentierten Produkte digital abgebildet und abgewickelt werden können.

Die Verifizierung der Authentizität einer Vielzahl von gemessenen ESR-Spektren kann vorteilhaft in einem gemeinsamen Nachweisschritt durchgeführt werden, beispielsweise unter Verwendung von sogenannten Zero-Knowledge-Proofs, welche die Authentifizierung einer Berechnung (beispielsweise einer Summe vieler Einzelwerte) ermöglicht, ohne den Einzelwert selbst offenbaren zu müssen. So kann die Authentizität einer Vielzahl von Produkten z.B. durch einen Dritten (Behörde, Dienstleister) in einem Arbeitsschritt verifiziert werden, ohne dass dem Dritten die einzelnen ESF-Fingerprint-Spektren und die damit codierte Information zur Kenntnis gelangen.

Der Verfahrensschritt der Messung des ESR-Spektrums eines zu authentifizierenden Produktes und der Erzeugung einer digitalen Repräsentation des gemessenen ESR-Spektrums kann in vorteilhafter Weise mit einem mobilen Endgerät, vorzugsweise einem Smartphone ausgeführt werden, auf welchem ein Computerprogramm ausgeführt wird, das unter Verwendung der Schaltkreiskomponenten des mobilen Endgerätes ein ESR-Spektrum des Produkts misst (sogenannte Software Defined Radio(SDR)-Schaltkreise). Dabei können zur Messung des ESR-Spektrums des Produktes ein zusätzlicher Permanentmagnet oder dazu geeignete Antennen oder externe zuschaltbare Schaltkreiskomponenten verwendet werden. Alternativ kann das ESR-Spektrum auch unter Verwendung des Erdmagnetfelds gemessen werden.

Die Erfindung schlägt ferner ein Verfahren zur Verifizierung der Authentizität eines Produktes aufweisend eine paramagnetische Phasen enthaltende Identifizierungs-Stoffzumischung vor, die ein ESR-Fingerprint-Spektrum aufweist, das eine eindeutige Identifizierung des Produktes erlaubt, aufweisend die Verfahrensschritte der Aufnahme eines ESR-Spektrums des Produktes mit einem mobilen Endgerät, vorzugsweise einem Smartphone, auf welchem ein Computerprogramm ausgeführt wird, das unter Verwendung der Schaltkreiskomponenten des mobilen Endgerätes ein ESR-Spektrum des Produkts misst, und des Vergleichs des aufgenommenen ESR-Spektrums des Produktes mit gespeicherten ESR-Fingerprint-Spektren.

Die vorliegende Erfindung schlägt außerdem eine Vorrichtung zur Authentifizierung eines Produktes aufweisend eine paramagnetische Phasen enthaltende Identifizierungs-Stoffzumischung vor, die ein ESR-Fingerprint-Spektrum aufweist, das eine eindeutige Identifizierung des Produktes erlaubt, wobei die Vorrichtung aufweist: eine Spektrometereinheit eingerichtet zur Messung eines ESR-Spektrums, eine Kommunikationseinheit eingerichtet zum Zugriff auf eine Datenbank, welche digitale Repräsentationen von ESR-Fingerprint-Spektrum speichert, und eine Datenverarbeitungseinheit eingerichtet zur Erstellung einer digitalen Repräsentation des gemessenen ESR-Spektrums des zu authentifizierenden Produktes und zum Vergleich mit den in der Datenbank gespeicherten digitale Repräsentationen von ESR-Fingerprint-Spektren.

Die Authentifizierungsvorrichtung ist vorzugsweise als mobiles oder stationäres Endgerät, etwa als Smartphone, ausgebildet, auf welchem ein Computerprogramm ausführbar ist, das unter Verwendung der Schaltkreiskomponenten des mobilen Endgerätes ein ESR-Spektrum des Produkts misst.

Die Erfindung schlägt schließlich ein auf einem Endgerät, vorzugsweise einem Smartphone, ausführbares Computerprogrammprodukt vor, welches, wenn es auf dem Endgerät ausgeführt wird, unter Verwendung der Schaltkreiskomponenten des Endgerätes ein ESR-Spektrum einer paramagnetische Phasen enthaltende Probe misst.

**Figurenbeschreibung**

Die Erfindung wird nachstehend anhand von Ausführungsbeispielen unter Bezugnahme auf die Zeichnungen im Einzelnen beschrieben. Dabei zeigt:

Fig. 1     ein schematisches Flussdiagramm eines Ausführungsbeispiels eines erfindungsgemäßen Verfahrens zur Produktauthentifizierung;

Fig. 2a    ESR-Spektren an Pulvergemischen aus UB und MAG in verschiedenen Gewichtsverhältnissen, aufgenommen bei Raumtemperatur;

Fig. 2b    die zweiten Ableitungen der ESR-Spektren aus Fig. 2a nach $H_{appl}$;

Fig. 3     eine schematische Illustration einer Messanordnung zur Produktauthentifizierung gemäß einem erfindungsgemäßen Ausführungsbeispiel;

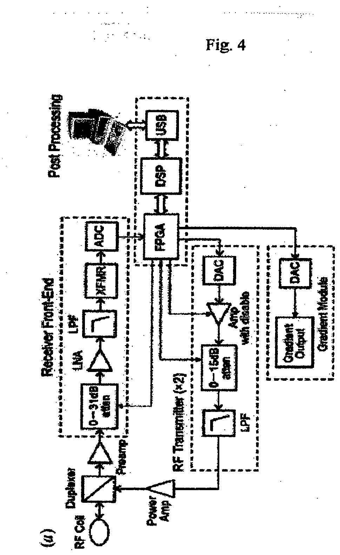

Fig. 4     das Blockdiagramm eines Single-Board-Schaltkreises zur Messung von Magnetoresonanzspektren;

Fig. 5     ESR-Spektren dünner Filme enthaltend $Fe_3O_4$, Ultramarin Blau, MAG und UB geschichtet;

Fig. 6     ESR-Spektren von a) Ultramarin Blau (UB), b) phen($CuCl_2$), c) UB und phen($CuCl_2$) im Gewichtsverhältnis 1:1 gemischt;

Fig. 7     ESR-Spektren von Magnetit bei verschiedenen Temperaturen;

Fig. 8     ESR-Spektren von Ultramarin bei 100 K bzw. Raumtemperatur;

Fig. 9    ESR-Spektren von Ultramarin und Magnetit als Tablette (a) und suspendierter Tablette (b);

Fig. 10    ESR-Spektren von verschiedenen Ultramarin Blau enthaltenden Extrudaten;

Fig. 11    ESR-Spektren von verschiedenen Ultramarin Blau enthaltenden Pasten;

Fig. 12    ESR-Spektren von verschiedenen TEMPO enthaltenden Pasten;

Fig. 13    ESR-Spektren von verschiedenen $TiO_2/SiO_2$ enthaltenden Pasten; und

Fig. 14    ESR-Spektren von verschiedenen dotiertes MgO enthaltenden Pasten.

Fig. 15    ESR-Spektren der Verbindungen und des Gemischs des Beispiels 11.

## Detaillierte Beschreibung der Erfindung

Der vorliegenden Erfindung liegt die überraschende Erkenntnis zugrunde, dass sich ESR-Spektren als Identifikationsmarker für eine große Vielzahl von Produkten besonders eignen, da

(a) ein ESR-Fingerprint-Spektrum durch Zumischungen zum Produkt selbst oder Identifizierung im Produkt selbst an Stelle von an- oder aufgebrachten Etiketten, Barcodes oder dgl. erzeugt wird, wodurch die Fälschungssicherheit und die Handhabbarkeit deutlich verbessert wird, die Nachweisempfindlichkeit ist dabei so gering, dass im Femtomolbereich detektiert werden kann und somit ein Umwelt- und funktionsbedingt unbedenklicher Marker, insbesondere wichtig im Nahrungs- und Pharmaziebereich, bereitgestellt werden kann. Überraschenderweise kann somit auch ohne einen aufwendigen Probenaufschluss die Probe identifiziert werden,

(b) ein ESR-Fingerprint-Spektrum durch verschiedene geeignete Herstellungs- und Beimischungsverfahren sowie Kombinationen mehrerer Verfahren dem Produkt gezielt hinzugefügt und damit beabsichtigte Information codiert werden kann,

(c) ein durch eine Kombination verschiedener Herstellungs- und/oder Beimischungsverfahren erzeugtes ESR-Fingerprint-Spektrum ohne das Wissen dieser Verfahren (Täterwissen) sehr schwierig kopierbar ist, wodurch wiederum die Fälschungssicherheit verbessert wird, und

(d) ein ESR-Spektrum zur Produktauthentifizierung mit vergleichsweise geringem apparativen und zeitlichen Aufwand aufgenommen werden kann.

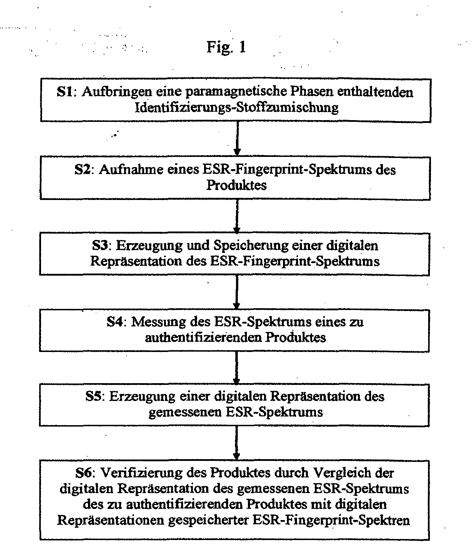

Das Flussdiagramm der Fig. 1 zeigt schematisch die einzelnen Verfahrensschritte eines Ausführungsbeispiels des erfindungsgemäßen Verfahrens zur eindeutigen Identifizierung und Authentifizierung eines Produktes.

Bei dem Produkt kann es sich beispielsweise um ein Arzneimittel, ein Lebensmittel oder ein Vor- oder Zwischenprodukt oder regulierter Bestandteil davon handeln, etwa eine Verpackung, Blister, Behälter, wie Glas- und Polymerröhrchen, eine Spritze, Ampulle oder ein Reservoir für Flüssigkeiten. Das Produkt kann aber auch ein Gewebe, ein Textil- oder Lederprodukt, eine Münze, ein Geldschein, ein Wertpapier, eine Urkunde, Zertifikat oder eine Scheck- oder Chipkarte oder ein Teil davon sein, oder ein einen Edel- oder Schmuckstein, ein Medizinprodukt, ein Implantat oder Transplantat, oder ein Ersatz- oder Zulieferteil für ein Industrieprodukt.

In einem ersten Verfahrensschritt S1 wird durch geeignete Verfahren eine paramagnetische Phasen enthaltenden Identifizierungs-Stoffzumischung auf oder in das Produkt auf/eingebracht. Diese Verfahren können dabei Beschichtung, Beimischung, Dotierung, Sputtering, chemische Radikalerzeugung, Bestrahlung, insbesondere Röntgenstrahlung, und/oder durch Druckverfahren umfassen. Letztere können dabei Hochdruck, Tiefdruck, Durchdruck, Siebdruck, Flachdruckwie Offsetdruck, Digitaldruck, Stahlstich, Siebdruck, Elektrofotographie (Laserdruck), Pulverdruckverfahren, wie Xerox und Elektrospray, Elektrospinning, Fällungen, Papierschöpfung oder Schichtverfahren wie etwa Siebdruck, oder Direct Imaging umfassen. Geeignete Rohstoffe und Medien dafür sind beispielsweise Pasten, Tinten, Beizen, Gase und Dämpfe, Lacke, Matrizen, Pulver, Lösungen, Schmelzen, Gläser, und physikalisch aktive bzw. chemisch reaktive Formen davon.

Im Verfahrensschritt S2 wird ein ESR-Fingerprint-Spektrum des so bearbeiteten Produktes aufgenommen und im anschließenden Verfahrensschritt S3 nach einer festgelegten Regel in eine digitale Repräsentation des ESR-Fingerprint-Spektrums umgewandelt und anschließend auf einem geeigneten Speichermedium wie einer Datenbank und oder einem verteilten Speichermedium (distributed ledger) wie etwa einer Blockchain abgespeichert.

Die Verfahrensschritte S1 bis S3 finden dabei typischerweise unter Kontrolle des Herstellers oder Erstvertreibers des fraglichen Produktes statt.

Die Authentifizierungs- bzw. Verifizierungsschritte S4 - S6 können beliebig oft durchgeführt werden, beispielsweise durch Zwischenhändler, Zollbehörden, Endverkäufer, Anwender, Nutzer, Kunden, etc.

Im Verfahrensschritt S4 wird das ESR-Spektrum eines zu authentifizierenden erfindungsgemäßen Produktes gemessen und dabei - wenn es sich um ein Originalprodukt handelt - das ESR-Fingerprint-Spektrum der Identifizierungs-Stoffzumischung erfasst. Das gemessene ESR-Spektrum wird in Verfahrensschritt S5 nach der festgelegten Regel digitalisiert und anschließend in Schritt S6 mit den digitalen Repräsentationen gespeicherter ESR-Fingerprint-Spektren des Herstellers /Lieferanten verglichen, um so die Authentizität des erfindungsgemäßen Produktes zu verifizieren.

Die Erfindung kann sinnvoll genutzt werden z.B. von Pharmaunternehmen, Produzenten, Lieferanten sowie Fachleuten entlang der Wertschöpfungskette bis hin zum Endkunden, z.B. Patient oder Sammler oder Endbesitzer.

In den folgenden Abschnitten werden verschiedene Aspekte der Erfindung näher erläutert.

**ESR-Spektren**

Es sind Systeme mit itinerantem Magnetismus bekannt, wobei Bandelektronen eine spontane magnetische Ordnung zeigen. Diese werden von solchen Systemen unterschieden, deren magnetische Ordnung durch lokalisierte Elektronenspins verursacht ist. Letztere sind in chemisch komplexierten Atomen, insbesondere fast allen farbigen Mineralien, oft als technische Füllstoffe und Pigmente bzw. Seltenen Erden bedeutsam. Andere wichtige paramagnetische Zentren sind Isolatoren, wie synthetische und natürliche Polymere und organische Farbstoffe, wie Chinoide, Anthocyane und Polyphenole.

Das magnetische Moment lokalisierter Elektronenspins wird aber auch in Haupt- und Nebengruppen mit steigender Ordnungszahl des chemischen Elements, das das dem lokalisierten Elektronenspin zugehörige Rumpfatom ist, zunehmend von Spin-Bahn Kopplungseffekten beeinflusst. Dem Materialwissenschaftlersind somit auch mikro- und makroskopische Spin-Gitter-Systeme bis hin zu metallischen Leiterkörpern bekannt.

Werden die genannten Systeme, ionische-atomare, chemische Komplexe, isolatorradikale wie Polymere, mineralisch inertierte bzw. natürlich mineralische, halbmetallische oder metallische, mit Mikrowellen bestrahlt, erhält man im allgemeinsten Sinne unterschiedliche stationäre oder dynamische Elektronenspinresonanz- bzw. ESR-Spektren.

Der ESR-Spektroskopie sind grundsätzlich nur Systeme mit ungepaarten Elektronen zugänglich, beispielsweise radikalische Systeme, paramagnetische Übergangmetalle, Bandmagnete und Halbleiter. In dem Aufsatz von Angelika Brückner in Chem. Ing. Tech. 2014, 86, 11, S. 1871-1882, ist dargelegt, dass, je nach System, der resonante Elektronenspin komplexen Wechselwirkungen unterworfen sein kann, zum Beispiel zwischen Elektronen- und Kernspin, und/oder von der räumlichen Symmetrie beeinflusst ist. Bei der Messung an Systemen aus mehreren überlagerten Komponenten verursacht dies komplexe, oft nicht einfach zu interpretierende ESR-Spektren. Zwar belegt dies das hohe Potential der spektroskopischen Methode für das Studium ungepaarter Elektronensysteme, lässt jedoch zugleich erkennen, dass eine Kombination verschiedener Systeme nicht einfach einer linearen oder einfach zu berechnenden Kombination von ESR-Spektren zugeordnet werden kann.

Verfolgt man z.B. eine gegebene Substanz bei ihrem Gang durch den menschlichen oder tierischen Organismus, kann man durch Erfassung von Ort, Identität und die zeitlicher Änderung des ESR-Spektrums Rückschlüsse auf die physikalische und /oder chemische Umwandlung des Aggregats oder der Substanz ziehen, beispielsweise während ihrer Auflösung beim Verdauungsprozess oder bei anderen Vorgängen der Metabolisierung.

Dorfman, J. Exp. Theor. Phys. 48 (1965), 715, schätzt ab, wie bei solchen Systemen makroskopische magnetische Observablen prinzipiell von der Korngröße abhängen. Insgesamt lässt sich bei hier relevanten Materialien, insbesondere der medizinisch technischen Formulierungen das Verhalten von Spinsystem, der das Moment gebenden "Sonde" des gesamten Aggregates sowie die legalistisch-regulatorische Anwendbarkeit schwer voraussagen. Die Intensität des ESR-Signals, gleichbedeutend mit dem Integral des Absorptionssignals, ist direkt proportional zur spontanen Magnetisierung $M_s$ der Probe, wie der Aufsatz von B. Heinrich und J.

F. Cochran in Advances in Physics 42 (1993), 523, darlegt. Die Linienbreite des ESR-Signals folgt einer Abhängigkeit in der Form von

$$\Delta H \sim K_1 / M_s$$

Wobei $K_1$ die magnetokristalline Anisotropiekonstante ist, vgl. Ya.G. Dorfman, J. Exp. Theor. Phys. 48 (1965), 715.

Auch die magnetische Formanisotropie hat einen wesentlichen Einfluss auf die Form und Position des ESR-Signals. Da die magnetokristallinen Anisotropiekonstanten der bekannten ferro- bzw. ferrimagnetischen Materialien im Bereich von 103- 106 $J/m^3$ liegen, wird dementsprechend eine ESR-Linienbreite

$$\Delta H \sim (10^2 \ldots 10^4)\ Oe$$

beobachtet. V.K. Sharma und F. Waldner im J. Appl. Phys. 48(1977), 4298, beobachteten die Linienbreite $\Delta H$ im ferrimagnetischen $Fe_3O_4$ Pulver von ~ 1000 Oe bei Raumtemperatur. Es ist anzumerken, dass die magnetokristalline Anisotropiekonstante von Magnetit etwa $3*10^4$ $J/m^3$ beträgt.

Es ist außerdem bekannt, dass in Partikeln mit einer bzw. unterhalb einer kritischen Größe thermische Fluktuationen über die magnetokristalline Anisotropie oberhalb einer kritischen Temperatur, auch Blocking-Temperatur genannt, dominieren, und so zeigen solche Partikel ein superparamagnetisches Verhalten. Unterhalb der Blocking-Temperatur weisen die Partikel dagegen ein ferro- bzw. ferrimagnetisches Verhalten auf. Die kritische Größe der Partikel wird durch magnetokristalline Anisotropie bestimmt. Im Magnetit liegt die kritische Partikelgröße bei etwa 14 nm, vgl. G. Vallejo-Fernandez et al., J. Phys. D: Appl. Phys. 46(2013), 312001. Magnetit-Nanopartikel mit Partikelgrößen von und unter 14 nm können relativ schmale ESR-Linien aufweisen, die für paramagnetische und superparamagnetische Partikel charakteristisch sind, diskutiert im Aufsatz von J. Salado et al., J. Non-Crystalline Solids 354 (2008), 5207, bzw. bei R. Berger, J. Magn. Magn. Mater. 234 (2001), 535. Eine besondere Form solcher Messungen ist die Erfassung paramagnetischer Einflüsse auf die bildgebende Kernspintomographie, deren Messung jedoch auf viel schwächeren Kernspin-Wechselwirkungen beruht.

Die Figur 1a zeigt beispielhaft ESR-Spektren an verschiedenen Gemischen von Magnetit $Fe_3O_4$ Pulver (MAG) und Ultramarin Blau (UB).

Bei einem gewichtsbezogenen Mischungsverhältnis von UB:MAG = 30 : 1 ist das ESR-Signal des $S_3$-Radikals bei g = 2,026 noch gut zu erkennen. Dies lässt darauf schließen, dass noch nicht alle $S_3$-Radikale des UB starke magnetische Dipolwechselwirkungen mit MAG eingegangen sind.

Doch bereits bei einem erhöhten Gehalt an MAG, entsprechend einem Gewichtsmischungsverhältnis UB:MAG = 30 : 3, erhielt man ein deutliches, breites ESR-Signal bei g = 2,307 aufgrund der ferrimagnetischen MAG Partikel. Das Signal der $S_3$-Radikale war dagegen wegen der starken magnetischen Wechselwirkung zwischen MAG und $S_3$-Radikal kaum noch zu erkennen. Bei einem auf ein Verhältnis von UB:MAG = 30 : 4 gesteigerten Gewichtsanteil an MAG fand man diesen Effekt weiter vergrößert.

Figur 1 b zeigt die zweite Ableitung dieser Linienformen nach dem für die Spektroskopie aufgewandten äußeren Magnetfeld $H_{appl}$. Die zweifach differenzierten Linienformen zeigten hier noch deutlicher das Radikalsignal, insbesondere beim Verhältnis UB:MAG = 30 : 4. Der mit dem MAG Anteil steigende Einfluss der magnetischen Wechselwirkungen zwischen MAG und UB macht sich in dem jeweiligen Peak-zu-Peak-Abstand in der zweifach nach dem Magnetfeld abgeleiteten Linienform bemerkbar.

## Gezielte Erzeugung charakteristischer ESR-Spektren

Ein Aspekt der vorliegenden Erfindung betrifft die gezielte Erzeugung bestimmter charakteristischer Eigenschaften eines ESR-Spektrums wie etwa einer Resonanzfrequenz oder der Linienbreite, welche Eigenschaften dann zur Codierung von Information (Produktcharakterisierung, Herstellungsort, Herstellungszeit, bestimmungsgemäßer Verwendungszweck, Rechte'etc.) verwendet werden kann. Die Erfindung umfasst verschiedene Kombinationen von Algorithmen, Datenarchitekturen und bekannten oder zukünftigen Erfassungs- und Verfolgungssystemen. Die Identifizierungs-Stoffzumischung kann entlang der gesamten Produktionskette und der Produktstufen aufgebracht werden. Überraschenderweise liegen die notwendigen Eigenschaften des spezifischen stabilen atomaren Magnetismus mit ausreichend großem magnetischen Moment in der Bandbreite der notwendigen physikalischen Auflösung und Empfindlichkeit und werden durch übliche Produktionsbedingungen nicht verändert und könnten vom Rohstoff bis zum nachgelagerten Transport und Verteilung in der Wertschöpfungskette angewendet und verarbeitet werden. Sehr überraschend sind die geeigneten Komponenten der erfindungsgemäßen Identifizierungs-Stoffzumischungen (etwa Dotierungen) für die meisten Anwendungen in den Listen der nicht aktiven Arzneimittel-Inhaltsstoffe und der regulierten umweltfreundlichen Pigmente und funktionellen Matrix- und Beschichtungsstoffe für viele Industrien zu finden, wie etwa polymerische Mineralien wie Silikate oder Salze und Oxide mit kleinen Gitteranomalien und Verunreinigungen.

Bei der Identifizierungs-Stoffzumischung kann es sich um eine paramagnetische Beschichtung, um paramagnetisch mineralischen, glasartigen, paramagnetisch molekulare oder paramagnetisch salzartig-ionische Füllerbestandteile in einer Polymer- oder Bindermatrix und einer Phase handeln, in der paramagnetische Zentren beispielweise durch ionisierende Bestrahlung oder chemische Reaktionen sukzessive erzeugt werden können, beispielsweise durch schichtweisen Prozessaufbau. Ein Auslesemagnetfeld richtet die elektronisch-paramagnetischen Zentren durch ein variables oder stabiles den Körper durchdringendes Magnetfeld aus und erzeugt die Energieniveaus, die mit Hilfe der Hochfrequenzeinheit eines ESR-Spektrometers wiederholt ausgelesen werden können. Dabei können die Resonanzsignale entweder additive Linien sein oder sie wechselwirken je nach Kombination charakteristisch und gezielt miteinander (Kopplung), was die Vielfalt der Markierungsarten, die Markierungsvarianten, bedeutend erweitert.

Andere physikalische Magneton-Wechselwirkungen können durch Ionisierung etwa mittels ionisierender Strahlung erzeugt werden. Die Veröffentlichung "Electron Spin Resonance Shift and Linewidth Broadening of Nitrogen-Vacancy Centers in Diamond as a Function of Electron Irradiation Dose" von E. Kim et al. beschreibt z.B. die gezielte Verschiebung einer ESR-Resonanzfrequenz und die Vergrößerung der Linienbreite bei Diamantproben durch Elektronenbestrahlung.

Einzelgewebe können unter Verwendung von Polymermatrizen effektiv mit einem ESR-Fingerprint-Spektrum versehen werden. Die ESR-Signale sind gut randomisiert, um eine gute Bit-Koaleszenz zu erhalten. Die Signale verändern sich nicht unter dem Einfluss von industriellen Formulierungen, Zubereitungen oder Produktionsprozessen bis hin zum Schmelzen von Polymeren und Gläsern. Die schichtweise Mischbarkeit der paramagnetische Phasen enthaltende Identifizierungs-Stoffzumischung ermöglicht eine praktisch uneingeschränkte Anwendung auf oder in Geweben von Polymerpulvern, Leder, Textilmaterialien oder Beschichtungen bis hin zu Glasverfahren, Extrusion oder additiven Herstellungsverfahren (3D-Druck).

**Messung der ESR-Spektren**

Ein Vorteil der Erfindung liegt darin, dass ESR-Spektren heutzutage mit relativ geringem apparativem und zeitlichem Aufwand gemessen werden können. Insbesondere ist das Signal-Rausch-Verhältnis wesentlich besser als bei aus der medizinischen Diagnostik bekannten Kernspiriresonanzmessungen (NMR).

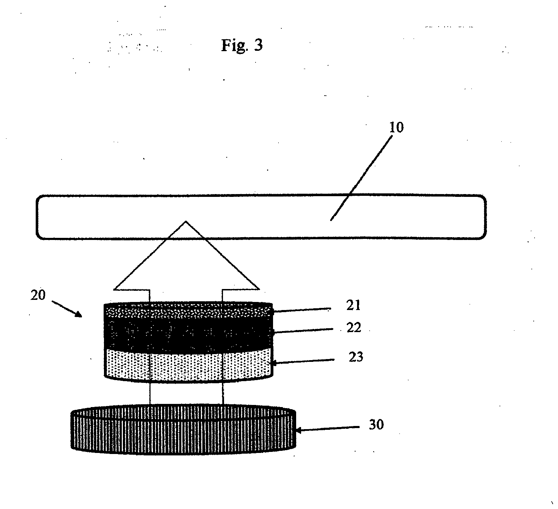

Die Veröffentlichung W. Tang und W. Wang, Meas. Sci. Technol. 22 (2011), 1-8 beschreibt ein auf einer einzigen Leiterplatte untergebrachtes NMR-Spektrometer mit einer durch Software definierten Funktionalität ("single-board software defined radio (SDR) spectrometer"). Die in dem Artikel vorgestellte Schaltungsanordnung ist in Fig. 3 wiedergegeben. Die SDR-basierte Architektur, realisiert durch die Kombination eines einzelnen Field Programmable Gate-Array (FPGA) und einen digitalen Signalprozessor (DSP) mit RF-Frontend-Schaltungen, macht das Spektrometer kompakt und rekonfigurierbar. Der DSP, der als Pulsprogrammierer arbeitet, kommuniziert mit einem Personal Computer über eine USB- Schnittstelle und steuert das FPGA über einen parallelen Port. Das FPGA führt digitale Verarbeitungsschritte aus wie ein numerisch gesteuerter Oszillator (NCO), digitaler Abwärtswandler (DDC) und ein Gradientenwellenformgenerator. Der NCO, mit agiler Steuerung von Phase, Frequenz und Amplitude, ist Teil eines direkten digitalen Synthesizers, der zum Erzeugen eines RF-Pulses verwendet wird. Der DDC führt eine Quadraturdemodulation, mehrstufige Tiefpassfilterung und Verstärkungsanpassung durch, um ein Bandpasssignal erzeugen (Empfängerbandbreite von 3,9 kHz bis 10 MHz). Der Gradientenwellenformgenerator ist in der Lage, geformte Gradientenimpulswellen auszugeben und unterstützt eine Wirbelstromkompensation. Das Spektrometer erfasst direkt ein NMR-Signal bis zu 30 MHz bei der Basisbandabtastung und ist für Low-Field (<0,7 T) -Anwendungen geeignet.

Da ESR-Signale ein wesentlich besseres Signal-Rausch-Verhältnis aufweisen als NMR-Signale, ist ein Auslesen von ESRSpektren erst Recht mit geringerem apparativem Aufwand, insbesondere mit einem mobilen Endgerät wie einem Smartphone durch Aufspielen einer geeigneten Softwareanwendung unter Ausnutzung der in dem Gerät schon vorhandenen elektronischen Bauelemente ("software defined radio SDR") möglich.

Fig. 3 zeigt in schematischer Darstellung ein Ausführungsbeispiel einer derartigen erfindungsgemäßen Vorrichtung zur Authentifizierung eines erfindungsgemäßen Produktes. Auf ein mobiles Endgerät 10 ist eine SDR-Softwareanwendung geladen, die unter Verwendung eines Magneten oder magnetisierbaren Bauteils 30 ein ESR-Spektrum der Identifizierungs-Stoffzumischung 20 eines erfindungsgemäßen Produktes erfasst. Bei der Identifizierungs-Stoffzumischung 20 kann es sich um eine paramagnetische Beschichtung 21, paramagnetisch mineralische, glasartige, paramagnetisch molekulare oder paramagnetisch salzartig-ionische Füllerbestandteile in einer Polymer- oder Bindermatrix 22 oder einer Phase 23 handeln, in der paramagnetische Zentren beispielweise durch ionisierende Bestrahlung oder chemische Reaktionen erzeugt sind. Das magnetische oder magnetisierbares Bauteil 30 richtet die elektronisch- paramagnetischen Zentren durch ein variables oder stabiles den Körper durchdringendes Magnetfeld H aus (senkrechter Pfeil)) und erzeugt die Energieniveaus, die mit Hilfe der Hochfrequenzeinheit eines ESR-Spektrometers oder eines SDR-Aufbaus in dem mobilen Endgerät 10 wiederholt ausgelesen werden kann. Dabei können die Resonanzsignale entweder additive Linien sein oder sie wechselwirken je nach Kombination charakteristisch und gezielt miteinander (Kopplung), was die Vielfalt der Markierungsarten, die Markierungsvarianten, bedeutend erweitert. Das Magnetfeld H erzeugende Bauteil 30 kann im Endgerät 10 selbst untergebracht sein oder ein Teil der Umgebung der Messsituation sein (z.B. Erdmagnetfeld). Feld-Sweep und Fix-Feld-Geräte können heute leicht miniaturisiert werden. Impulshöhe (Energie) wird dabei durch das H-Feld vordefiniert. Es kann leistungssparend durch Permanentmagneten oder durch Feldspulen erzeugt und verändert werden. Auch Pulsfolgenteile können dort überlagert werden. Außerdem können separate Hochfrequenz-Sende- und Empfangsspulen die Pulse geben und FID-Anteile aufnehmen. Die Geometrie dieser Hochfrequenzkopfes kann miniaturisiert werden und wird durch das Objekt oder die erfindungsgemäße paramagnetisch markierte Oberfläche bestimmt.

Die obengenannte Veröffentlichung von Tang and Wang beschreibt detailliert eine solche Messapparatur für NMRKernspins; Verfahren, die physikalischquantenmechanisch vergleichbar mit ESR sind, bei denen aber um Größenordnungen geringere Spin-Magnetische Momente gemessen werden.

Die notwendigen Analog-Digitalkonverter und die Leistungstreiber sowie die gesamte Hochfrequenzelektronikwird besonders rauscharm gehalten, da direkte Signaltsynthesizerarchitektur verwendet wird, indem ein "Single Field Programmable Gate Array" (FPGAs)-Chip ein programmierbares "Software Defined Radio" (SDR) in unmittelbarer Nähe zum Multiplexer der kleinen Hochfrequenzspule bildet. Also entfallen praktisch alle Rauschanteile durch analoge Signalmischung und Filterung, die digital-mathematisch durchgeführt werden. Die eigentliche Überraschung und Revolution hat allerdings im Smartphone-Bereich dazu geführt, dass Analog-DigitalKonverter und sogar FPGA-Chips, beispielsweise das ICE5LP4K-Chip im iPhone 7 mit breitester Möglichkeit der Programmierung zur Emulierung eines SDRs des erfindungsgemäßen Systems mit Datenbankzugang und weltweiter Konnektivität, in Echtzeit oder bei Anbindung an Netzte ermöglichen. Es sind bereits heute in diesen Feldgeräten eine Vielzahl von Baugruppen verfügbar, um die notwendigen Pulse, Gleichstromsteuerung und HF- FID-Akquisition digital aufzunehmen und mathematisch-digital zu filtern und aufzubereiten.

Dies bedeutet, dass selbst vor einer speziellen Hardware-Anpassung durch etwa spezielle "ASIC"-Komponenten (custom-designed chips) die SDR-Philosophie moderner Smartphones in der Lage ist, mit existierenden Treibern und den "DSP"-Chips (Digital Signal Processing) das programmierbare ESR-Lesegerät für erfindungsgemäße Authentifizierung mit geringster Peripherie (z.B. Permanentmagnet, Abschirmung, Hochfrequenzspule, Feldspule, Leistungstreiber) durch Aufspielen von "Apps" (Verwendungsprogrammen) erlauben.

## Blockchain-Netzwerke

Wie das Internet die Veröffentlichung, Speicherung, und Verbreitung von Information revolutioniert hat, ist die Blockchain-Technologie dabei, die Speicherung und Übertragung von Werten wie Geld, Unternehmensanteilen, etc. zu revolutionieren. Blockchain-Netzwerke bestehen aus einer Vielzahl miteinander verbundener Netzwerkknoten, deren gemeinsamer Status über ein Konsensusprotokoll dezentral aktualisiert und in einer Kette miteinander verketteter Blöcke ("Blockchain") in Form kryptographischer Codes gespeichert wird. Das über den Inhalt der Blöcke bestimmende Konsensusprotokoll kann wie bei bitcoin auf einem sogenannten Proof-of-Work-Protokoll basieren, bei dem mehrere sogenannte Miner im Wettbewerb ein kryptographisches Rätsel lösen (Hash-Werte kleiner als ein bestimmter Schwellenwert zu erzeugen), um den nächsten Block zu erzeugen und die damit verbundene Prämie (im Jahre 2019 12,5 Bitcoin) zu verdienen. Um an der nächsten Runde im Wettbewerb teilzunehmen, müssen die Netzwerkknoten zunächst die Validität des aktuellen Blocks bestätigen, wodurch der Konsens über den Status des Netzwerks hergestellt ist. Alternativ zum Proof-of-Work-Protokoll gibt es auch Proof-of-Stake-Protokolle, bei denen der Konsens über den Status des Netzwerks nicht durch den Einsatz von (stromfressender) Rechenleistung, sondern durch die Hinterlegung von werthaltigen Netzwerktoken mittels eines probabilistischen Verfahrens erzielt wird. Daneben gibt es Mischformen sowie geschlossene (private) Blockchain-Netzwerke, bei denen autorisierte Netzwerkknoten den Netzwerkstatus bestimmen. Insbesondere auf dem Proof-of-Work-Konsensusprotokoll basierende öffentliche Blockchain-Netzwerke zeichnen sich durch ein hohes Maß an Manipulationssicherheit aus, da zur Veränderung zeitlich zurückliegender Ereignisse die gesamte dazwischenliegende "Historie" der Blockchain durch extrem rechenintensives Nacharbeiten des Konsensmechanismus neu erschaffen werden müsste. Durch die dezentrale Speicherung des Netzwerkstatus auf einer Vielzahl von Netzwerkknoten ("distributed ledger") besteht auch eine hohe Sicherheit gegen Datenverlust, es gibt keinen "single point of failure".

Auf einem Blockchain-Netzwerk können materielle Güter (Produkte, Sendungen, Ersatzteile, Rohstoffe, Arzneimittel und Medikamentenlose) oder immaterielle Güter wie Nachrichten, Währungsbeträge oder Geschäftsanteile repräsentiert und gehandelt werden. Durch den für alle transparenten, gemeinsamen Netzwerkstatus wird das Risiko des Ausfalls einer Vertragspartei ("counterparty risk") verringert, das für die Abwicklung einer Transaktion erforderliche Vertrauen in die Gegenpartei wird reduziert und durch Vertrauen in den mathematischen und spieltheoretischen Konsensusalgorithmus ersetzt.

Verschiedenste Typen derartiger Blockchain-Netzwerke werden mittlerweile eingesetzt, um Waren bei physischen Standortwechseln (Lager, Vertrieb, Kunde) oder Transaktionen (z.B. Eigentümerwechsel) z.B. einer Kühlkette zu verfolgen und abzuspeichern. Idealerweise ist eine lückenlose Rückverfolgbarkeit der individuellen Provenienz einer einzelnen Einheit, einer Verpackung, eines Pakets von Verpackungen bis zum Ursprung des Endprodukts und sogar über ihre Vorläufer, Pasten, Fertigmischungen und Rohstoffquellen hinaus möglich.

Das Netzwerk kann weiterhin ein sicherer Aufbewahrungsort für geheim - oder z.B. stochastisch erzeugtes Produktionswissen sein, wenn diese Daten verschlüsselt z.B. in Form von Hashwerten auf der Blockchain abgespeichert werden. Die Authentizität der Daten kann dann durch Vergleich des auf der Blockchain mit Zeitstempel gespeicherten Hashwertes mit einem aus den privaten, vertraulichen Daten erzeugten Hashwert nachgewiesen werden.

Um die auf dem Blockchain-Netzwerk gespeicherte Realität mit der physischen Realität eines hergestellten oder verarbeiteten Produktes abzugleichen, stellt bislang die Produktverpackung eine wesentliche Schwachstelle dar. Smart Tags wie RFIDs müssen am einzelnen Produkt angebracht und entfernt werden, benötigen Gehäuse und Befestigungsmechanik. Dieses Problem wird durch die erfindungsgemäße paramagnetische Phasen enthaltende Identifizierungs-Stoffzumischung gelöst, die ein eindeutig produktidentifizierendes ESR-Fingerprint-Spektrum aufweist, welches nicht von einer Produktverpackung oder dgl. abhängt. Dabei kann das Fingerprint-ESR nur sehr schwierig gezielt verändert werden, ähnlich wie der Eintrag in einem Blockchain-Ledger. Die Erfindung stellt so ein eindeutiges und manipulationssicheres Verzeichnis ("ledger") von Produkten der physischen Welt dar, als Pendant des verteilten manipulationssicheren Verzeichnisses, welches Blockchain-Netzwerke bereitstellen.

## Beobachtung chemischer Umwandlungsprozesse

Das erfindungsgemäße Produkt kann beispielsweise als Körper zur Aufnahme im menschlichen oder tierischen Organismus ausgestaltet sein.

Die Erfinder sind vollkommen überraschend auf einen weiteren Zusammenhang gestoßen. Hat man im bisherigen Kenntnisstand ein ESR-Spektrum als typisch für die bestrahlte Substanz aufzufassen, so lässt sich beispielsweise das technische Problem lösen, wie systematisch kontrollierte und gewollte Umwandlungsprozesse der Substanz, insbesondere durch Kombinationen verschiedener Systeme, etwa in Form von Gemischen, Verbindungen oder allgemein Zusammensetzungen aus verschiedenen makroskopischen oder mikroskopischen Phasen für die jeweilige Zusammensetzung charakteristische ESR-Spektren liefern. Es wurden Zusammensetzungen aus zumindest zwei Materialien gefunden, bei denen zumindest ein Material außerhalb der Zusammensetzung in seiner reinen Form ein charakteristisches ESR-Spektrum liefern würde. Doch in der Zusammensetzung mit zumindest einem weiteren Material ist ebendieses ESR-Spektrum überraschend stark abgeschwächt oder vollkommen verschwunden. Dabei weist ein Körper, der mehrere Phasen aufweist und durch den menschlichen oder tierischen Organismus aufgenommen wird oder sich innerhalb des Organismus befindet, zumindest zwei Phasen mit einem unterschiedlichen Elektronenspin-Resonanzspektrum auf. Zumindest eine der Phasen weist itineranten oder lokalisierten Magnetismus auf. ESR-Spektren Seltener Erden werden weniger gut unterdrückt gefunden, wobei, je nach Kombination, der Körper eine Abschwächung des ESR-Spektrums bzw. eine Überlagerung verschiedener ESR-Spektren zeigt.

Es kann vorteilhaft sein, wenn zumindest eine Phase des erfindungsgemäßen Körpers rein paramagnetische Zentren aufweist, bevorzugt S-Radikale, vorzugsweise ausgewählt aus Ultramarin. Es kann besonders vorteilhaft sein, anstatt des Ultramarins superparamagnetische Partikel auszuwählen, vorzugsweise enthaltend oder bestehend aus Magnetit oder Maghemit oder Pyrite oder eisenhaltige Verbindungen, wie Amethyst. Bei solchen Partikeln wird ein ähnliches ESR-Signal gefunden.

Vorzugsweise weist zumindest eine Phase des erfindungsgemäßen Körpers zumindest einen kollektiv ordnenden Zustand auf, welcher ferro-, ferri-, und/oder antiferromagnetisch sein kann. Besonders bevorzugt weist diese EisenSauerstoffVerbindungen auf. Ganz besonders bevorzugt ist zumindest eine Phase Magnetit oder eine Phase aus dem Fe-O System. Die erwähnten Phasen sind insbesondere für den menschlichen oder tierischen Organismus unschädliche Substanzen. Außerdem können derart ausgewählte Phasen als Tablettenformulierung ausgeprägt sein. Überraschend sind die Größenordnungen des ESR-Spektrum abschwächenden oder unterdrückenden Effektes.

Die Phasen können des Weiteren in Partikeldispersionen nachgestellt werden. Wiederum ist überraschend, dass damit auf einfache Art und Weise eine pharmazeutische Formulierung bereitgestellt werden kann, denn gerade Magnetit oder ein Material mit Fe-O Phasen ist für den menschlichen Organismus sehr gut verträglich und wäre auch in der Humanmedizin außerordentlich sicher in der Anwendung. Der Körper ließe sich somit ebenfalls im gastrointestinalen Bereich sicher einsetzen, weil der Körper weder hochgiftige Substanzen noch schädliche Radikale aufweist.

Bei jeglicher Spektroskopie werden umso bessere Messresultate erzielt, je besser das Signal-Rausch-Verhältnis des betrachteten Systems ist, wobei das System in diesem Falle der betrachtete Organismus mit dem erfindungsgemäßen Körper und der Instrumentierung zur Erfassung des ESR-Spektrums ist. Menschliche und tierische Organismen zeigen in Magnetfeldern bei Weitem überwiegend diamagnetisches Verhalten, und diamagnetischer Hintergrund stört selbst die viel empfindlichere Kernspintomographie kaum. Also sind beim Einsatz des erfindungsgemäßen Körpers für die Ausmessung der ESR Spektren nur sehr geringe Magnetfeldstärken erforderlich.

Weiterhin kann es vorteilhaft sein, dass bei dem erfindungsgemäßen Körper zumindest eine Phase umhüllt ist von zumindest einer weiteren Phase. Besonders bevorzugt umhüllt eine Phase als dünner Film eine weitere Phase. Vorzugsweise können die Dicke des Films und die Phasen derart ausgewählt sein, dass das ESR-Spektrum der inneren, umhüllten Phase durch das ESR-Spektrum der äußeren, umhüllenden Phase vollkommen verdeckt ist.

Geht der Durchgang des erfindungsgemäßen Körpers durch den menschlichen oder tierischen Organismus mit der Zersetzung des Körpers einher, so tritt mit der Zersetzung der umhüllenden Phase das ESR-Signal der umhüllten Phase zeitabhängig stärker werdend zutage. Diese einfache Zeitabhängigkeit ist eine weitere vorteilhafte Eigenschaft des Körpers.

Falls man Magnetit-Partikel in zumindest einer Phase des Körpers auswählt, sind die Erfinder der Ansicht, ohne an eine bestimmte Theorie gebunden zu sein, dass das ESR Spektrum nicht nur durch intrinsische magnetische Eigenschaften, sondern auch durch Dipol-Wechselwirkungen zwischen Magnetit-Partikeln verursacht werden könnte. Die Wechselwirkungen werden vorzugsweise von der Form der Partikel, zum Beispiel Kugel, Nadel, Kubus, allgemein von der räumlichen Verteilung des Magnetits, bspw. Film, beeinflusst. Diese Formen zeigen unterschiedliche Entmagnetisierungsfelder.

Je mehr ferri- bzw. ferromagnetische Anteile der erfindungsgemäße Körper aufweist, desto stärker wird das ESR-Signal geschwächt. Hierbei wird eine Absorption der bei der Spektroskopie eingestrahlten Mikrowellen vermutet.

Es sind weiterhin Körper denkbar, in denen eine ferromagnetische und eine radikalische Phase, zum Beispiel eine Ultramarin-Phase, räumlich getrennt vorliegen, vorzugsweise in Form räumlich getrennter Ballungen. Dem entspricht ein eindeutiges ESR-Spektrum. Wird der Körper nun zersetzt, passiert eine vorübergehende Durchmischung beider Phasen, und bei geeignetem Mengenverhältnis der einen zu der anderen Phase verschwindet das ESR-Spektrum einer Phase, vorzugsweise die des Ultramarin, zeitweise ganz. Somit kann die Zersetzung des Körpers im Organismus spezifisch dem Zersetzungsprozess zugeordnet werden.

Es kann weiterhin vorteilhaft sein, wenn der erfindungsgemäße Körper zumindest drei Phasen aufweist, wobei eine Phase vorzugsweise paramagnetisch ist, vorzugsweise ausgewählt aus (phen)CuCl$_2$. In diesem Falle ist die ESR-Linienform komplexer, und man erhält ein zeitaufgelöstes Verhalten beim Zersetzen der Mischung der Phasen, beispielsweise beim Zersetzen des Körpers während des Stoffwechsel-Prozesses im Organismus, das mit einer Zeitabhängigkeit des ESR-Spektrums nachgewiesen wird. Es ist eine fortschreitende Zersetzung dokumentierbar.

Demnach können vorzugsweise magnetische, paramagnetische und radikalische Phasen kombiniert werden. Wird ein derart zusammengesetzter Körper im Organismus zersetzt, erscheint mit dem zersetzungsbedingten Verschwinden der magnetischen Phase oder deren Ablösung vom Körper eine andere, sogenannte "finale" ESR-Linienform, die sich von der ESR-Linienform des unzersetzten erfindungsgemäßen Körpers deutlich unterscheidet.

Solche Zersetzungsvorgänge sind bei nicht therapeutischen Vorgängen vorteilhaft, wie zum Beispiel im Rahmen persönlicher, nicht medizinisch motivierten Fragen der Ernährung oder Ernährungsgewohnheiten.

Die Zersetzungsvorgänge sind jedoch auch Ziel von beispielsweise medizinischen Implantaten, bei deren funktionalen Beschichtungen und besonders oraler Darreichungsformen von nutrazeutischen, diätetischen beziehungsweise therapeutischen Formulierungen wie z.B. Kapseln, Tabletten, Filme und Granulate und multipartikulären Darreichungsformen der Lebensmitteltechnologien und, davon unabhängig, Arzneimitteltechnologen. Sie können sehr gezielt durch die Wahl der eingesetzten Exzipienten z.B. der Kapselhüllen, Partikelcoatings und der eingesetzten Materialien der Medizintechnik designed und damit durch den Formulierungsprozess gesteuert werden. Bevorzugt sind dabei Löslichkeit, besonders bevorzugt pH- und zeitabhängige Löslichkeit solcher Hilfsstoffe und Exzipienten im Einsatz. Bei medizinischtechnischen Implantaten ist es besonders die Hydrolyse, die zu gewünschter Resorption von Matrizes und Beschichtungen führt. Beispielsweise genannt sind die zulässigen Materialien und Polymere Eudragit®-Methacrylate und Resomer®-Polyester, modifizierte Stärken wie HMPC, HMPC-AS oder Polylactite und Co-Glycolite oder Co-Caprolacton für chirurgisches Material, sowie resorbierbare medizintechnische Beschichtungen oder Implantate. Dabei können solche Isolatorpolymere, insbesondere medizinisch-technischen Polymere selbst paramagnetische Zentren tragen, wie sie z.B. bei der Sterilisationsbestrahlung mittels e-Beam- oder $\gamma$-Bestrahlung entstehen. Somit ist es weiterhin bevorzugt, dass der erfindungsgemäße Körper zumindest eine Phase mit zumindest einem medizinisch-technischen Polymer aufweist, das ein paramagnetisches Zentrum, vorzugsweise isolierte Radikale aufweist.

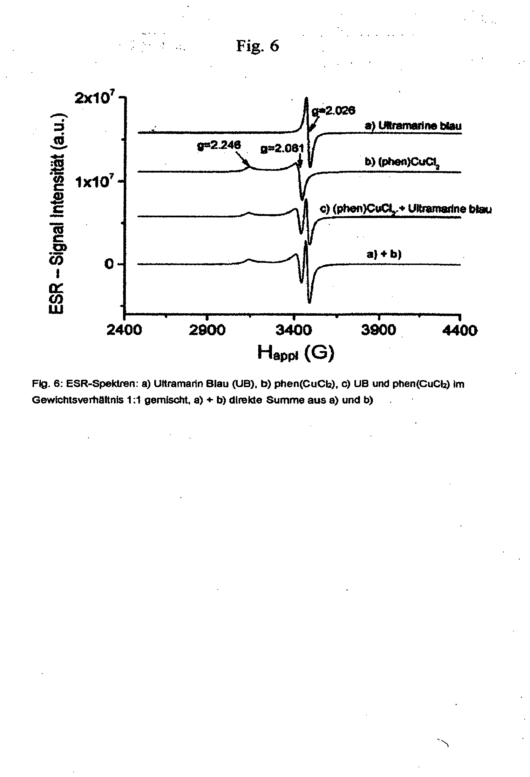

Somit kann das Erscheinen der finalen ESR-Linienform als Fingerprint des Körpers während der Zersetzung im Organismus aufgefasst werden. Dies wird in Beispiel 2 und Figur 6 näher erläutert werden.

Da also Mischphasen von Reinphasen unterscheidbar sind bzw. der Entfall zumindest einer Phase des erfindungsgemäßen Körpers detektierbar ist, können nun auch Dosierungen, gleichbedeutend Mischungen verschieden aufgebauter Körper im Organismus detektiert werden.

Also ist gleichfalls Gegenstand von Ausführungsbeispielen der Erfindung die Verwendung des erfindungsgemäßen Körpers, der vorzugsweise zumindest drei Phasen aufweist, für das Monitoring von Zersetzungsvorgängen im menschlichen oder tierischen Organismus.

**Beispiele**

Die Erfindung wird im Folgenden anhand von Beispielen näher erläutert. Unter dem Begriff "Raumtemperatur" wird im Rahmen der Erfindung eine Umgebungstemperatur von ca. 20 °C verstanden.

Beispiel 1 :

Lose Pulver-Vormischungen erlauben eine einfache Änderung während des Herstellungsverfahrens bis hin zu einer eindeutigen Identifizierungs-Stoffzumischung für einzelne Tabletten oder Kleinteile, die mit anderen Verfahren, mikroelektronischen Komponenten oder physischen Etiketten nur schwer realisierbar ist.

Die Bestandteile einer solchen Pulver-Vormischung können dabei sein:

1) Mikrokristalline Cellulose oder HPMC
2) Vestamid oder PVP
3) PEEK
4) Eudragit
5) PLA

| Nr | Standard | Paramagnetisches Zentrum |
|----|----------|--------------------------|
| 1 | Cu/Al$_2$O$_3$ | Single Cu(II) |
| 2 | Mn/MgO | Single Mn(II) |

(fortgesetzt)

| Nr | Standard | Paramagnetisches Zentrum |
|---|---|---|
| 3 | $TiO_2$ oder $ZrO_2$ | F-Zentrum |
| 4 | $C_3N_4$ | Signal von Leitungsbandelektronen |
| 5 | Ultramarin | $S_3^-$ |

Folgende Varianten sind dabei denkbar:

a) Alle Oxide gemischt, verdünnt in Hilfsstoff 1
b) Oxidmischung ohne die Pigmente der stärksten zwei Signale, verdünnt in Hilfsstoff 1
c) Pressling von a (z.B. aus IR-Presslingpresse)
d) b, aber verdünnt in Hilfsstoff 2
e) Hilfsstoff 3 bestrahlt (Kontrolle)
f) Hilfsstoff 4 bestrahlt (Kontrolle)
g) Hilfsstoff 5 bestrahlt (Kontrolle)
h) b, verdünnt in Hilfsstoffs, 4 oder 5 (je nach Erfolg) bestrahlt
i) b, verdünnt in Hilfsstoffs, 4 oder 5 (je nach Erfolg) nicht bestrahlt
j) Alle Oxide gemischt in Hilfsstoff 3, 4 oder 5 (je nach Erfolg), Referenz zu h und i
k) Eine weitere Verdünnung in Hilfsstoff 1 wie a (so verdünnt sodass gerade noch detektierbar, z.B. 1:100)
l) Falls Hilfsstoff 3, 4 oder 5 bestrahlt funktioniert haben, zwei von diesen gemischt mit zwei Oxiden entsprechend verdünnt
m) Mit Wasser feuchte Paste einer der obigen Gemische
n) Geschmolzene Mischung (stellen wir erkaltet zur Verfügung mit Ultramarin)

Bei der Anwendung als Laminat oder Extrusion können dabei regulatorische Aspekte bezüglich maximaler Konzentrationen bestimmter Stoffe berücksichtigt werden.

Beispiel 2: Erfindungsgemäßer Körper enthaltend Ultramarin Blau und Magnetit

Magnetit $Fe_3O_4$ Pulver, im Rahmen der Erfindung mit "MAG" abgekürzt, Handelsname "Cathay pure Black B2310", erhältlich bei Cathay Industries, und Ultramarin Blau Pulver, abgekürzt mit "UB" oder "Ultramarin", Handelsname "Kremer Pigment, Product Nr. 45000", wurden unter Verwendung von Mörtel mit Hilfe eines Stößels in den Gewichtsverhältnissen MAG:UB = 1 : 30, 3 : 30 bzw. 4 : 30 gemischt.

An dem so erhaltenen Gemisch wurden ESR-Spektren im X-Band (9,5 GHz) bei Raumtemperatur und einer Mikrowellenenergie von 6,3 mW, bei einer Modulationsfrequenz von 100 kHz und einer Amplitude bis zu 5 Gauß aufgenommen.

Des Weiteren wurden auf verschiedene Klebestreifen jeweils dünne Schichten enthaltend MAG, wobei die Konzentration des MAG zusätzlich mit Methylzellulose verdünnt war, bzw. UB aufgebracht, wobei zuvor jede dieser Komponenten in Form einer Suspension in Ethanol bereitgestellt worden war. An den so erhaltenen Schichten wurden ESR-Spektren aufgenommen.

Um sich zu vergewissern, dass UB und MAG einen innigen Kontakt eingegangen waren, so dass eine genügend große Wechselwirkung mit dem $S_3^-$-Radikal vorlag, wurden die ESR-Spektren zunächst an separaten dünnen Schichten aufgenommen. Anschließend wurde das ESR-Spektrum jeweils an den aufeinander geklebten Klebestreifen erfasst.

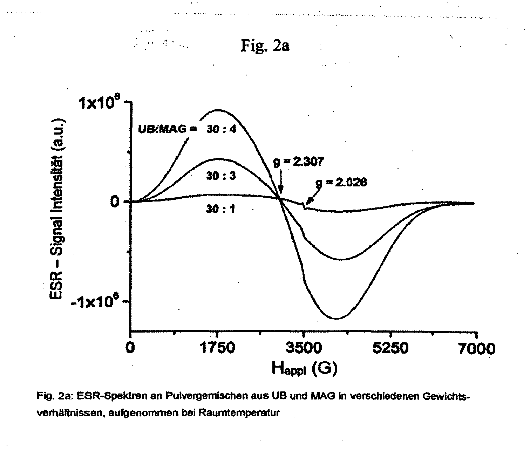

Die Fig. 2a zeigt ESR-Spektren an verschiedenen Gemischen von MAG und UB. Bei einem gewichtsbezogenen Mischungsverhältnis von UB:MAG = 30 : 1 ist das ESR-Signal des $S_3^-$-Radikals bei g = 2,026 noch gut zu erkennen. Dies lässt darauf schließen, dass noch nicht alle S3-Radikale des UB starke magnetische Dipolwechselwirkungen mit MAG eingegangen sind.

Doch bereits bei einem erhöhten Gehalt an MAG, entsprechend einem Gewichtsmischungsverhältnis UB:MAG = 30 : 3, erhielt man ein deutliches, breites ESR-Signal bei g = 2,307 aufgrund der ferrimagnetischen MAG Partikel. Das Signal der $S_3^-$-Radikale war dagegen wegen der starken magnetischen Wechselwirkung zwischen MAG und $S_3^-$-Radikal kaum noch zu erkennen. Bei einem auf ein Verhältnis von UB:MAG = 30 : 4 gesteigerten Gewichtsanteil an MAG fand man diesen Effekt weiter vergrößert.

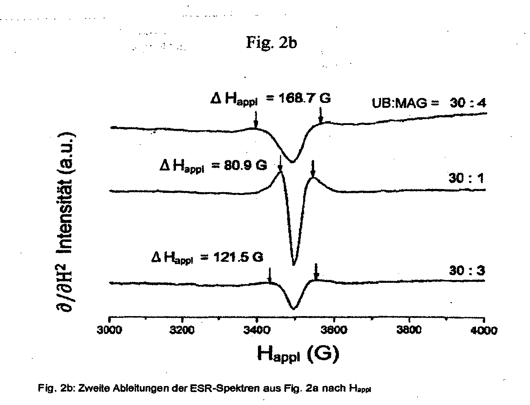

Die zweite Ableitung dieser Linienformen nach dem für die Spektroskopie aufgewandten äußeren Magnetfeld $H_{appl}$ lieferte das Diagramm in Figur 2b. Die zweifach differenzierten Linienformen zeigten hier noch deutlicher das Radikalsignal, insbesondere beim Verhältnis UB:MAG = 30 : 4.

Der mit dem MAG Anteil steigende Einfluss der magnetischen Wechselwirkungen zwischen MAG und UB machte sich in dem jeweiligen Peak-zu-Peak-Abstand in der zweifach nach dem Magnetfeld abgeleiteten Linienform bemerkbar.

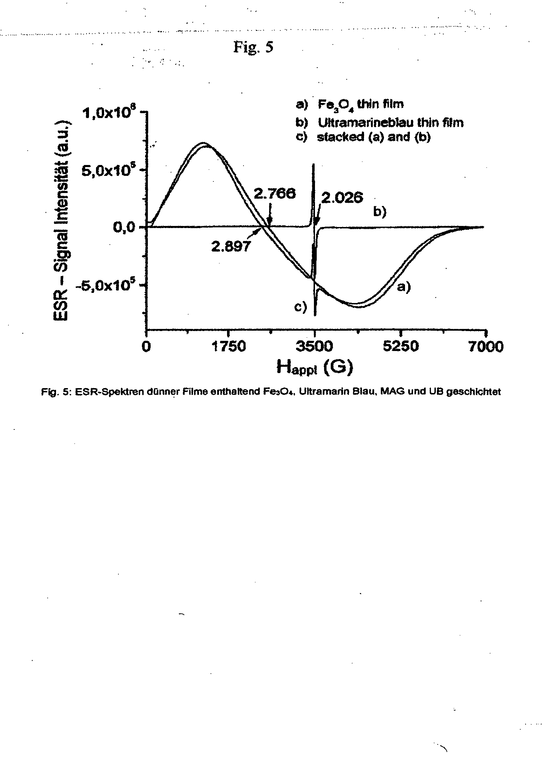

Fig. 5 zeigt ESR-Spektren, die an dünnen Schichten aus UB und MAG auf Klebestreifen erhalten wurden.

Erwartungsgemäß stimmten die ESR-Signale der Schichten mit MAG bzw. mit UB mit den ESR-Signalen der reinen Komponenten MAG bzw. UB überein.

Wurde jedoch eine innige Verbindung durch das Kleben der Klebestreifen aufeinander bereitgestellt, wurden andere ESR-Signale erhalten.

Die Intensität des vom $S_3$-Radikal bewirkten ESR-Signals fand man abgeschwächt, wohingegen das ESR-Signal des MAG kaum an Intensität verlor, dafür jedoch eine leichte Verschiebung von einem Wert von g = 2,766 auf g = 2,897 erfahren hatte. Es wird angenommen, dass dieser Effekt der magnetischen Dipolwechselwirkung zwischen MAG und UB zuzuschreiben war, was vermutlich bedeutet, dass bereits das mechanische Inkontaktbringen der dünnen Schichten auf den Klebestreifen das ESR Signal des $S_3$-Radikals und das ferromagnetische ESR-Signal simultan beeinflusst hat.

Die eben demonstrierten ESR-Spektren zeigen, dass in Gemischen von UB und MAG ein Anteil MAG von bereits etwa 10 Gew. -% ausgereicht hat, um das ESR-Signal des $S_3$-Radikals unter die Nachweisgrenze zu drücken. Sogar das Inkontaktbringen dünner Schichten enthaltend beide Komponenten schwächte dieses Signal auf etwa den halben Wert.

Wurde dagegen ausschließlich eine paramagnetische Komponente mit UB gemischt, erhielt man das $S_3$-Radikal ESR-Signal beinahe unverändert, und zwar selbst dann, wenn der Anteil der paramagnetischen Komponente viel höher war als der von MAG.

Ohne an eine bestimmte Theorie gebunden zu sein, vermuten die Erfinder die Ursache der Verschiebung im ESR-Signal in der Fig. 5 in dem magnetischen Zustand der Partikel, der eine Selbstentmagnetisierung verursacht. Das resultierende interne Feld $H_{int}$ kann mit einer einfachen Beziehung approximiert werden:

$$H_{int} = H_{appl} - N\,M,$$

wobei M die Magnetisierung, N ein Entmagnetisierungsfaktor und $H_{appl}$ das für die Spektroskopie äußere aufgewandte Magnetfeld ist. Die Entmagnetisierung ist von der Geometrie der M aufweisenden Partikel oder Substanz sowie der globalen Form des Körpers, welcher aus solchen Partikeln oder Substanz besteht, abhängig. In Form einer Schicht beispielsweise, die auf das Spektrum in Fig. 5 geführt hat, findet man ein deutlich stärkeres Entmagnetisierungsfeld, wenn das äußere Magnetfeld senkrecht zur Schichtoberfläche angelegt wird, als durch sphärische oder kubische Partikel bzw. Körper bewirkt ist. Dabei kann N in der Nähe von 1 vermutet werden. Bei sphärischen oder kubischen Partikeln bzw. Körper, die insbesondere nicht als Schicht angeordnet sind, kann N ~ 1/3 angesetzt werden. Auch steht zu vermuten, dass das entmagnetisierende Feld die Verschiebung der ESR-Spektren durch eine Änderung von magnetostatischer Wechselwirkung verursacht, wenn die Schichten enthaltend von Magnetit und Ultramarin aufeinander gestapelt sind, als die oben erwähnten Dipol-Wechselwirkungen in dem Falle, dass Magnetit und Ultramarin zusammen gemischt sind.

Beispiel 3: Körper enthaltend Phen(CuCl$_2$) und Ultramarin Blau

Wie Beispiel 2, jedoch wurde das Gemisch anstelle mit MAG mit paramagnetischem Dichloro(1,10-phenanthroline)Cu$^{II}$ (Phen(CuCl$_2$)) Komplex und Ultramarin Blau im Gewichtsverhältnis 1:1 bereitgestellt.

Wurde im Beispiel 2 ein erheblicher Abschwächungseffekt beobachtet, der in der starken magnetischen Wechselwirkung zwischen MAG und dem $S_3$-Radikal Anion des Ultramarin Blau begründet ist, war diese Wechselwirkung zwischen der paramagnetischen Komponente mit Cu$^{II}$ Ionen (d$^9$, Spin = 1/2), nämlich dem Phen(CuCl$_2$) Komplex, nicht vorhanden.

Das ESR-Spektrum des paramagnetischen phen(CuCl$_2$) Komplex zeigte die typischen Signale des Cu$^{II}$ bei g = 2.246 und g = 2.061, gezeigt in der Fig. 6, Linienform b). Aus der Mischung mit UB erhielt man das ESR Spektrum als Überlagerung des Cu$^{II}$ und des $S_3$- Radikals (Fig. 6, Linienform c)). Offenbar stimmte die Linienform c) in sehr guter Näherung mit der direkten Summe der Linienformen a) und b) überein, siehe Fig. 6, Linienform a) + b). Dies belegt eine verschwindende magnetische Wechselwirkung zwischen Cu$^{II}$ und $S_3$- des Ultramarin Blau.

Beispiel 4: Erfindungsgemäßer Körper als Tablette in Wasser suspendiert

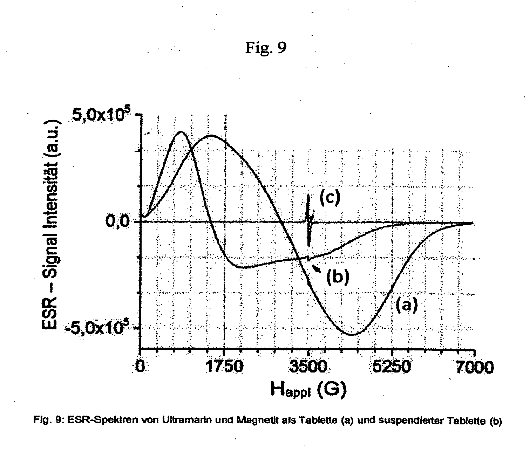

Es wurde eine Mischung von 10 mg Fe$_3$O$_4$, 10 mg Ultramarineblau und 130 mg Methylcellulose zu einer Tablette gepresst, indem die Mischung während 2 min einem Druck von 10 bar ausgesetzt wurde. Die so erhaltene Tablette wurde zerkleinert und in einem Becherglas in Wasser suspendiert. Für die ESR-Messungen wurden nach unterschiedlichen Zeiten Proben der Suspension in eine Glaskapillare gefüllt. Es wurden in Abhängigkeit von der Zeit verschiedene ESR-Spektren erhalten, die in Figur 9 gezeigt sind, und zwar mit der Linienform (a) die noch nicht suspendierte Tablette und mit der Linienform (b) das Signal der Tablette nach fortgeschrittener Suspension.

Die erkennbare Gesamtintensität des ESR-Signals belegt den mit der Zeit veränderten Gehalt an suspendiertem Feststoff. Das erfindungsgemäße Monitoring von Zersetzungsprozessen ist somit auch für ein einfaches Auflösen des erfindungsgemäßen Körpers möglich. Die Linienform (c) in der Fig. 9 zeigt zum Vergleich das Magnetit freie ESR-Signal.

Vergleichsbeispiel: ESR-Messungen an reinem Magnetit bzw. Ultramarin

An jeweils einer festen Probe Magnetit, Handelsname "Cathey Pure Black B2310 (40969)", und einer Probe Ultramarin, Handelsname "Kremer Pigment (45000)", wurden ESR-Spektren im X-Band bei unterschiedlichen Temperaturen aufgenommen.

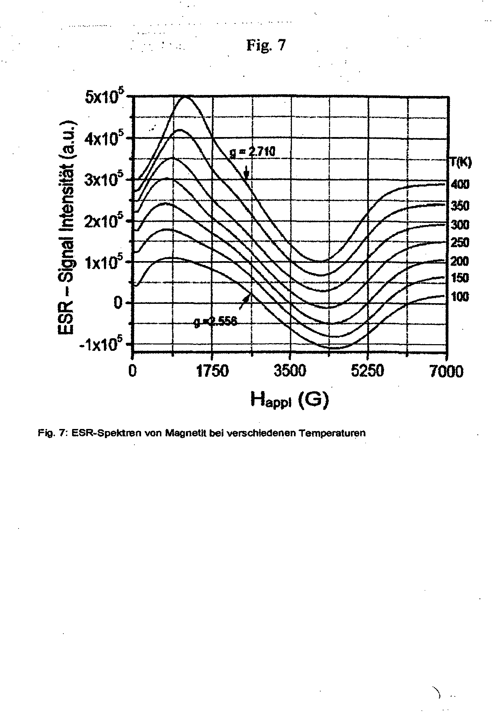

Der reine Magnetit zeigte das typische breite asymmetrische Singulett für ferromagnetisches Verhalten, dessen Linienform sich mit steigender Temperatur reversibel änderte, gezeigt in Fig. 7.

Ein solches Verhalten ist vermutlich auf eine Überlagerung ferromagnetischer Domänen unterschiedlicher Struktur und/oder Orientierung zurückzuführen.

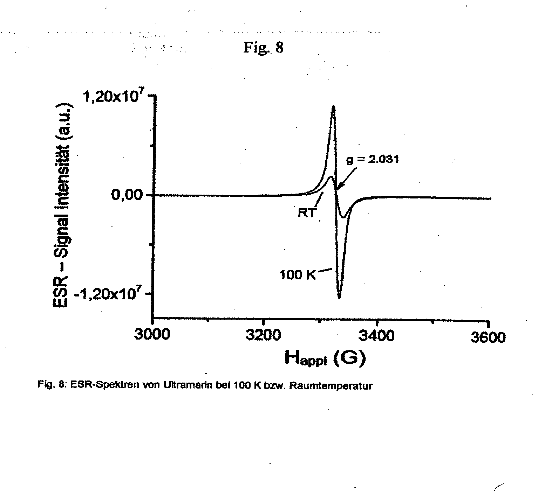

Das ESR-Spektrum des Ultramarins enthielt ein schmales isotropes Signal, das dem $S_3$-Radikal zuzuordnen war, siehe Fig. 8. Es wurde das für rein paramagnetische Zentren typische Temperaturverhalten beobachtet, d. h., die Intensität stieg mit sinkender Temperatur.

Beispiel 5: Erfindungsgemäße, Ultramarin Blau enthaltende Extrudate

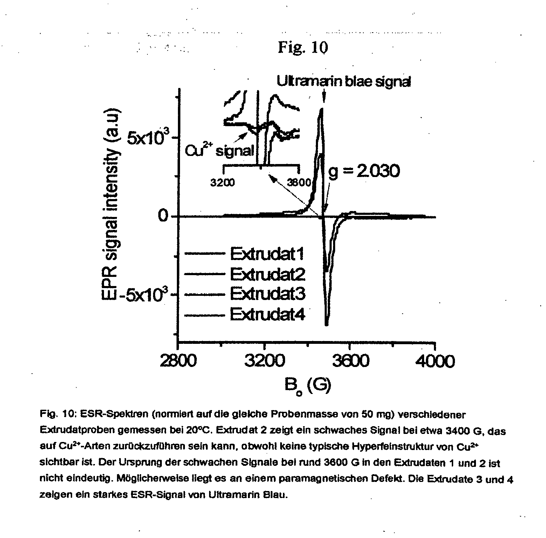

Verschiedene, Ultramarin Blau enthaltende Extrudate werden hergestellt und die ESR-Spektren gemessen. Fig. 10 zeigt die Spektren verschiedener Extrudatproben gemessen bei 20°C, normiert auf die gleiche Probenmasse von 50 mg. Extrudat 2 zeigt ein schwaches Signal bei etwa 3.400 G, das auf $Cu^{2+}$-Arten zurückzuführen sein kann, obwohl keine typische Hyperfeinstrukturvon $Cu^{2+}$ sichtbar ist. Der Ursprung der schwachen Signale bei rund 3600 G in den Extrudaten 1 und 2 ist nicht eindeutig. Möglicherweise liegt es an einem paramagnetischen Defekt. Die Exrudate 3 und 4 zeigen ein starkes ESR-Signal von Ultramarinblau.

Beispiel 6: Erfindungsgemäße, Ultramarin Blau enthaltende Pasten

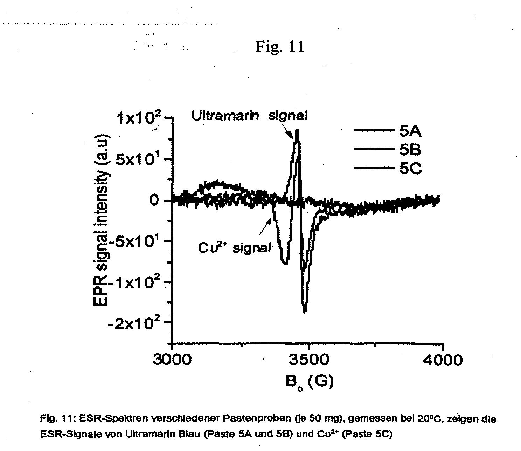

Fig. 11 zeigt die ESR-Spektren von drei verschiedenen Pastenproben von je 50 mg, gemessen bei 20°C. Pasten 5A und 5B zeigen die ESR-Signale von Ultramarin Blau, Paste 5C das Signal von $Cu^{2+}$.

Beispiel 7: Erfindungsgemäße, TEMPO enthaltende Pasten

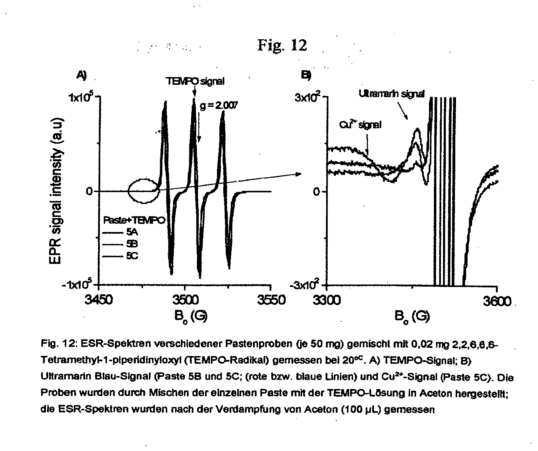

Fig. 12 zeigt die ESR-Spektren verschiedener Pastenproben (je 50 mg) gemischt mit 0,02 mg 2,2,6,6-Tetramethyl-1-piperidinyloxyl (TEMPO-Radikal), gemessen bei 20°C. A) TEMPO-Signal; B) Ultramarin Blau-Signal (Paste 5B und 5C, und $Cu^{2+}$-Signal (Paste 5C). Die Proben wurden durch Mischen der einzelnen Paste mit der TEMPO-Lösung in Aceton hergestellt, die ESR-Spektren wurden nach der Verdampfung von Aceton (100 µL) gemessen.

Beispiel 8: Erfindungsgemäße, $TiO_2/SiO_2$ enthaltende Pasten

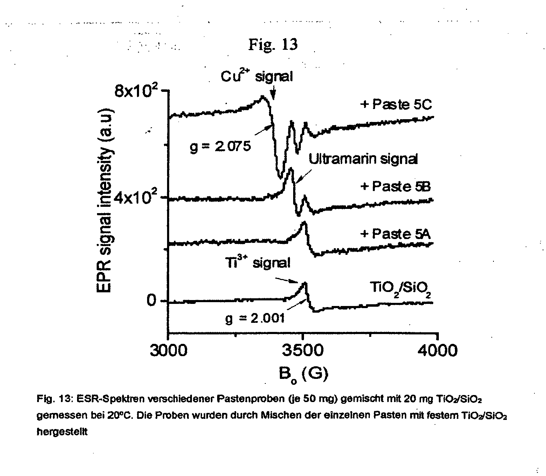

Fig. 13 zeigt ESR-Spektren verschiedener Pastenproben (je 50 mg) gemischt mit 20 mg $TiO_2/SiO_2$, gemessen bei 20°C. Die Proben wurden durch Mischen der einzelnen Pasten mit festem $TiO_2/SiO_2$ hergestellt.

Beispiel 9: Erfindungsgemäße, dotiertes MgO enthaltende Pasten

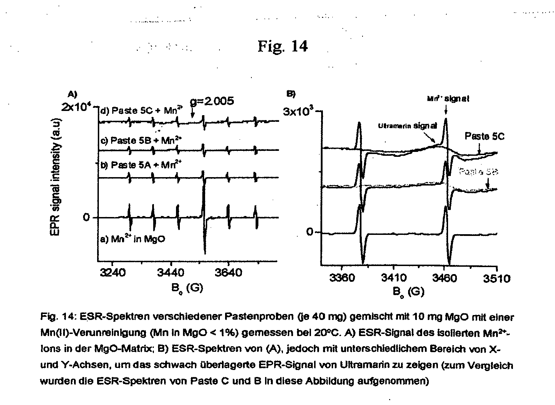

Fig. 14 zeigt ESR-Spektren verschiedener Pastenproben (je 40 mg) gemischt mit 10 mg MgO mit einer Mn(II)-Verunreinigung (Mn in MgO < 1%), gemessen bei 20°C. A) ESR-Signal des isolierten $Mn^{2+}$-Ions in der MgO-Matrix; B) ESR-Spektren von (A), jedoch mit unterschiedlichem Bereich von X- und Y-Achsen, um das schwach überlagerte EPR-Signal von Ultramarin zu zeigen. Zum Vergleich wurden die ESR-Spektren von Paste C und B in diese Abbildung aufgenommen.

Für die folgenden Beispiele 10 bis 12 wurden ESR-Messungen der nachstehenden markierten Proben und Markierungsgemischen mit technisch relevanten Funktionen und zulassungsrelevanter toxischer und umweltrelevanter Unbedenklichkeit wie folgt durchgeführt:

Die Proben für ESR-Messungen können als feste oder flüssige Substanzen analysiert werden. Für feste Substanzen wurde ein Quarzglasprobenröhrchen mit entsprechender Probenmenge ca. 10 - 1000 mg (je nach Probenröhrchendurchmesser) verwendet. Für flüssige Proben wurde ein Volumen von ca. 50 µL eingesetzt, welches mittels Glaskapillare aufgezogen wurde. Die Glaskapillare wurde im Anschluss für die Messung in ein Quarzglasprobenröhrchen geschoben. Vor dem Vermessen der Proben wurde ein Aufwärmzyklus des Gerätes durchlaufen, welcher 15 min in Anspruch nimmt und eine Endtemperatur von ca. 31 °C erreicht. Der Temperaturbereich während der Messungen betrug 31 ± 2 °C. Das Probenröhrchen wurde anschließend in die Messzelle eingeführt (im Optimalfall befand sich der Mittelteil der Probe (mittlere Höhe der Probe im Probenröhrchen) in 62 mm Entfernung zum Probenhalter, welcher das Probenröhrchen am oberen Ende umschließt. Die Messungen erfolgten bei einem Modulationswert (modulation) von 0,2 mT und 10 mW Mikrowellenleistung (microwave power) für eine Messlaufzeit (sweep time) von 60 Sek. über den Bereich des Magnetfelds (magnetic field range) 0 bis 400 mT. Nach der Messung wurde das ESR-Spektrum auf mögliche charakteristische Ausschläge (Peaks) untersucht. Diese wurden durch ihre Position im Magnetfeld (position), ihre Intensität (intensity/height), ihre Breite (width), ihre Fläche (area), ihre individuelle Erscheinung und falls mehrere Ausschläge detektiert wurden über ihren Abstand zueinander (distance) charakterisiert. Die Position wird dabei üblicherweise entweder als magnetische Flussdichte (B) in Millitesla oder dimensionslos als sogenannter g-Faktor (g-factor) angegeben. Für die ESR-Messungen wurde das Messgerät MS-5000 (11-0185) von der Freiberg Instruments GmbH (Freiberg, Deutschland) verwendet. Der Einfachheit halber wurden die gemessenen Proben in (++) = Signal ohne vorherige Anregung (+) = Signal mit Anregung durch Röntgenstrahlen und (-) = kein Signal eingeteilt.

Eingesetzte und gemessene Verbindungen/Mischungen:

- Kupfer(II)sulfat (++) natürliches Signal, stabil in den meisten Matrizes
- Mangan(II)chlorid (++), ebenso, allerdings mehr redoxvariant, funktional
- Mangan(IV)oxid (++) sehr charakteristisches Koppelsignal, funktional
- Zirkonium(IV)Oxid (++)/(+) schwaches natürliches Signal, aktivierbar bei harter Röntgenstrahlung
- Lactose-Monohydrat (++)/(+) durch Röntgenstrahlung mit Strahlung Dosisquantitativ aktivierbar, leichte natürliche Signalkomponente
- HPMC (-) kein Signal, nicht aktivierbar durch Röntgenstrahlung
- HMPCAS (-), Matrix, kein Signal, nicht aktivierbar durch Röntgenstrahlung
- Titandioxid (++) natürlicher signifikant
- PVP (Kollidon 30) (-) kein Signal Matrix
- Eudragit® E, L, RL, FL 30 D (+) Signal nach Röntgenbestrahlung, zeigt typische Signalsättigung, nichtlinear pro Dosis
- natürliches Ultramarinblau (++), starkes signifikantes Signal, extrem robust, alle Pigmentmodifikationen und Korngrößen
- D(-)-Mannit (+) Signal nach Röntgenbestrahlung, zeigt Dosis-Linearität
- Diamantpulver (++) entstehungsbedingte Ortsdotierung, zeigt signifikante hohe Ortsauflösung im Gitter
- Magnesiumoxid (++) Signal, Pigment
- Tintenschwarz (Carbon Black und Additive) (++)/(+) Signal nach Röntgenbestrahlung, schwaches Grundsignal, funktional
- Druckerpapier weiß grundiert mit Titandioxid als Pigmentsignal (++)
- Druckerpapier mit lokaler Ultramarindotierung (++), Signal additiv zum Grundierungssignal
- D(+)-Trehalose (+) Signal nach Röntgenbestrahlung
- Mikrokristalline Cellulose (MCC; Avicel PH) (+) Signal nach Röntgenbestrahlung
- Polylactid-co-Glycolid (Resome®) (+), Signal nach Röntgenbestrahlung
- Protein (tripelhelicales Collagen) (+) charakteristisches Signal nach Röntgenbestrahlung
- Gemisch aus Lactose-Monohydrat, MCC, natürliches Ultramarinblau, Diamantpulver, Kupfer (II) Sulfat und Magnesiumoxid (Kodierungs-Blend).

Beispiel 10

Das vorstehend beschriebene Kodierungs-Blend wurde anfänglich und später verdünnt gemessen. Dabei wurde ein Gemisch aus natürliches Ultramarinblau, Diamantpulver, Kupfer (II) Sulfat und Magnesiumoxid in äquivalenten Massen hergestellt. Zu diesem Gemisch wurden dann Lactose-Monohydrat und MCC (in äquivalenten Massen Lactose-Monohydrat und MCC zueinander) gegeben. Für das anfängliche Kodierungs-Blend wird das Gemisch zu Lactose-Monohydrat + MCC in 2/3 : 1/3 Gewichtsteilen eingesetzt. Danach werden Kodierungs-Blends mit einem höheren Anteil an Lactose-Monohydrat + MCC, hergestellt, wobei das Gemisch verdünnt auf ½, ¼ und ⅛ der anfänglichen Konzentration wird.

Die Spektralkurven zeigten den additiven Signaleffekt über den gesamten überstrichenen Magnetfeldbereich mit der höchsten Intensität bei 4,5 % (Masse/Masse) verdünnt auf 2,25 %, 1,125 % bis zu einer Matrix-Verdünnung 0,5625 %, der niedrigsten Intensitätskurve, was noch weit über das Signalrausch-Verhältnis bei dieser Methode von ca.

10 relativen Intensitätseinheiten als Rauschgrenze liegt. Überraschenderweise, ohne besondere Reinheitsanforderungen des Labors und der Substanzen, sind als Einzelpunktmessung solche Gemische und solche Verdünnungen so signifikant als Markersignal verwertbar bis zu einer Verdünnung von 0.0080 %, durch Verwertung der Gesamtspektreninformation sogar weit darunter.

Beispiel 11

Mit Röntgenstrahlung aktivierte Proben

Bestrahlung der Proben:

Ein Aliquot von 1 g Probe wurde jeweils abgewogen und anschließend mittels Röntgenstrahlung aus einer Rhodiumröntgenröhre bestrahlt. Hierzu wurde das Röntgenfluoreszenzspektrometer Axios DY1402 (Malvern Panalytical [0135] GmbH, Kassel, Germany) verwendet. Die Stromstärke betrug 66 mA und die Spannung 60 kV. Die Proben wurden somit einer Leistung von 3960 W ausgesetzt. Die Bestrahlzeit betrug 20, 30 beziehungsweise 40 Sek. Folgende Verbindungen wurden getestet:

- Lactose-Monohydrat (20s, 30s, 40s)
- PVP (Kollidon 30) (20s, 30s, 40s)
- Eudragit® L 100 (20s, 30s, 40s)
- Diamantpulver (20s, 30s, 40s)
- Ink (black, white) (40s)
- D(-)-Mannit (20s, 30s, 40s)
- HPMC (20s, 30s, 40s)
- Zirconium(IV)oxid (20s, 30s, 40s)
- Titandioxid (20s, 30s, 40s)
- Polylactid-co-Glycolid (20s, 30s, 40s)
- Protein (tripelhelicales Collagen) (20s, 30s, 40s)

Die Einzelverbindungen Lactose-Monohydrat und D(-)-Mannit zeigen bei dieser Strahlungsdosis und Wellenlänge eine neue, nach Dosis in weitem Bereich lineare Resonanzintensität. Die Spektren sind signifikant und die Substanzen können beliebig gemischt werden. Als Zucker sind sie geeignet für Nahrungsmittel und pharmazeutische Produkte. Titanoxid dagegen verändert sein Spektrum durch Bestrahlung, somit kann es auch erfindungsgemäß funktionell als innerer Marker verwendet werden. Auch Titandioxid ist unbedenklich und als Füllsubstanz und Pigment weit verbreitet. Mit der Energie-Impulshöhe der Rhodiumquelle wird dagegen bei Zirkon, PVP und Diamantpulver keine Spektrenveränderung, wie bei anderen Polymeren detektier. Eudragit® L 100 zeigt die besondere Eigenschaft, dass nach Röntgenbestrahlung schnell eine quantitative Signalsättigung erzeugt wird (Beschrieben). Dieser Effekt (Innerer Standard) wird vermutlich durch eine untergeordnete Polymer-Produktkomponente erzeugt die bei Bestrahlung quasi quantitativ in den ESR-sichtbare Modifikation gehoben wird. Solche funktionellen Besonderheiten sind markant, spezifisch und damit für die erfindungsgemäße Markierung funktionell erwünscht. Auch HPMC als Cellulose basiertes Polymer wird bei dieser Methode noch nicht ESR-sichtbar teilmodifiziert (Beschrieben). Ein minimales Signal in der Größenordnung der Rauschgrenze der nicht optimierten Methode zeigt jedoch die Möglichkeit einer intrinsischen Markierung durch Bestrahlung.

Beispiel 12

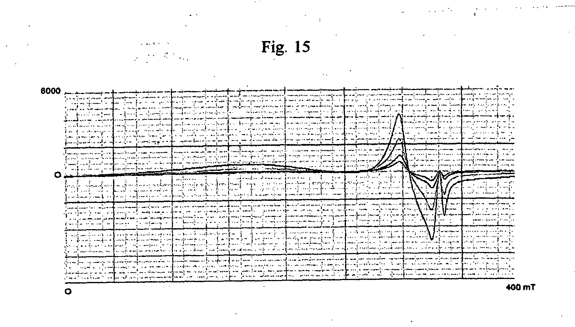

Zuletzt wurden alle hier verwendeten signalgebenden Gemische und vermessenen Einzelsubstanzen des Beispiels 11 in einer einzigen Darstellung vereint (Figur 15). Dies verdeutlicht die breite Verwendung des technisch zur Verfügung stehenden Spektralbereichs (Energiebereiche der Resonanzen, hier wie üblich angegeben (magnetische Flussdichte, B in mT). Auch die Signalbreite ist je Marker extrem unterschiedlich und kann zur Spezifität der Signalerkennung Verwendung finden. Die Signale sind in einem sehr breiten Dynamikbereich signifikant über Rauschen und können automatisch, durch Spektrensimulation, Spektren-Principle Component Analyse und durch Lernroutinen (Neuronal/Deep Learning Mustererkennungs-/ und Künstliche Intelligenzmethoden, Fingerprint Peak-Picking B, B-frequenznormierter Modus, g-Faktor-Normierung) leicht in allen hier verwendeten Verdünnungen typisiert und identifiziert werden. Der signifikante aber schwache Spektralanteil des MgO (Magnesium(II)oxid) wird dabei von den Methoden erkannt, wo das Auge versagt, d.h. Grundlinienauflösung und Basislinienkorrekturen sind für diese neuen statistisch-mathematischen Methoden nicht notwendig. Die Methode ist sowohl in Anforderung der Prozesse, Reinheit und Umgebungsbedingungen als auch im dynamischen Bereich quantitativ unerreicht.
Die durchgeführten Versuche zeigen bei dieser Spektrensimulation aus tatsächlich gemessenen Einzelspektren der oben genannten Verbindungen und bestrahlten Probenmodifikationen ohne Spezialmethode und ohne jede Probenvorbereitung eine Dynamik bis in den Pikogramm-Bereich der Signalerkennung. Die Kanalspezifität, die durch die signifikanten Signalformen gegeben sind, lassen eine Code-Kanal-Tiefe von mindestens 50-100 im Sinne von Mess-Dimensionen (Charakteristische Merkmale oder Stellen des "Bar"-Codes) erkennen. Die Messungsempfindlichkeit hier war ohne Spezialroutine Pikogramm pro Gramm Probe. Mit der charakteristischen Merkmalsbreite und einfachen Erhöhung der Messmethode in Sende-Leistung- und Signalakquisition ist die Methode im Femtogramm-Bereich breit anwendbar.

Insbesondere ist die Methode anwendbar in Bereichen wo Material-, Gesundheits- und Umwelteigenschaften in keiner Weise beeinträchtigt werden.


**Patentansprüche**

1. Produkt, aufweisend eine paramagnetische Phasen enthaltende Identifizierungs-Stoffzumischung, die ein ESR-Fingerprint-Spektrum aufweist, das eine eindeutige Identifizierung des Produktes erlaubt, **dadurch gekennzeichnet, dass** das ESR-Fingerprint-Spektrum in Form einer digitalen Repräsentation vorliegt, die fälschungs- und manipulationssicher auf einem Blockchain-Netzwerk abgespeichert ist.

2. Produkt gemäß Anspruch 1, wobei die paramagnetischen Phasen der Identifizierungs-Stoffzumischung gebildet werden durch eine oder mehrere Phasen ausgewählt aus:

   - paramagnetischen Zentren, bevorzugt S-Radikale, vorzugsweise ausgewählt aus Ultramarin,
   - kollektiv ordnenden Zuständen ausgewählt aus ferro-, ferri-, und/oder antiferromagnetischen Zuständen, vorzugsweise ausgewählt aus Eisen-Sauerstoff-Verbindungen, besonders bevorzugt Magnetit oder einem Material mit Fe-O Phasen,
   - einem Polymer, das ein paramagnetisches Zentrum, vorzugsweise isolierte Radikale aufweist,
   - molekularen Paramagneten, und
   - paramagnetischen Phasen mineralischer Stoffe und/oder Salze, insbesondere paramagnetische Phasen von $Al_2O_3$, $SiO_2$, natürlich oder künstlich dotiertem Diamant oder $ZrO_2$.

3. Produkt gemäß Anspruch 1 oder 2, wobei die digitale Repräsentation des ESR-Fingerprint-Spektrums einen aus dem ESR-Fingerprint-Spektrum abgeleiteten Hash-Wert enthält.

4. Produkt gemäß einem der Ansprüche 1-3, wobei das Produkt ein Pharmazeutikum (Arzneimittel), ein Lebensmittel oder ein Vor- oder Zwischenprodukt oder regulierter Bestandteil davon ist, beispielsweise eine Verpackung, ein Blister oder ein Behälter oder wobei das Produkt ein Gewebe, ein Textil- oder Lederprodukt, eine Münze, ein Geldschein, Wertpapier, Urkunde, Zertifikate oder eine Scheck- oder Chipkarte oder ein Teil davon, ein Edel- oder Schmuckstein, ein Medizinprodukt, ein Implantat oder Transplantat, oder ein Ersatz- oder Zulieferteil für ein Industrieprodukt ist.

5. Produkt gemäß einem der Ansprüche 1-4, wobei das ESR-Fingerprint-Spektrum einen Hersteller, einen Herstellungsort, eine Herstellungszeit, und/oder das Produkt selbst oder produktionsspezifische Daten, wie bestimmungsgemäßer Verwendungszweck oder Rechte codiert.

6. Verfahren zur Herstellung eines eindeutig identifizierbaren Produktes gemäß einem der Ansprüche 1-5, umfassend den Verfahrensschritt des Auf- oder Einbringens an/auf/in das Produkt einer paramagnetische Phasen enthaltenden Identifizierungs-Stoffzumischung, die ein ESR-Fingerprint-Spektrum aufweist, das eine eindeutige Identifizierung des Produktes erlaubt, und Erzeugung und Speicherung einer digitalen Repräsentation des ESR-Fingerprint-Spektrums fälschungs- und manipulationssicher auf einem Blockchain-Netzwerk.

7. Verfahren zur Produktauthentifizierung, aufweisend die Verfahrensschritte:

   (a) Auf- oder Einbringen einer paramagnetische Phasen enthaltenden Identifizierungs-Stoffzumischung an/auf/in ein Produkt oder Identifizieren eines Produktes, welches eine paramagnetische Phasen enthaltende Identifizierungs-Stoffzumischung enthält, welche Identifizierungs-Stoffzumischung ein ESR-Fingerprint-Spektrum aufweist, das eine eindeutige Identifizierung des Produktes erlaubt,
   (b) Aufnahme eines ESR-Fingerprint-Spektrums des Produktes,
   (c) Erzeugung und Speicherung einer digitalen Repräsentation des ESR-Fingerprint-Spektrums,

(d) Messung des ESR-Spektrums eines zu authentifizierenden Produktes und Erzeugung einer digitalen Repräsentation des gemessenen ESR-Spektrums,

(e) Verifizierung des zu authentifizierenden Produktes durch Vergleich der digitalen Repräsentation des gemessenen ESR-Spektrums des zu authentifizierenden Produktes mit digitalen Repräsentationen gespeicherter ESR-Fingerprint-Spektren.

8. Verfahren gemäß Anspruch 7, wobei die digitale Repräsentation eines ESR-Fingerprint-Spektrums einen aus dem ESR-Fingerprint-Spektrum abgeleiteten Hashwert enthält.

9. Verfahren gemäß Anspruch 7 oder 8, wobei die einem Produkt zugeordneten digitalen Repräsentationen der ESR-Fingerprint-Spektren fälschungs- und manipulationssicher auf einem Blockchain-Netzwerk abgespeichert werden, wobei vorzugsweise für jede abgespeicherte digitale Repräsentation eines ESR-Fingerprint-Spektrums ein eindeutiger non-fungible-token auf dem Blockchain-Netzwerk erzeugt wird.

10. Verfahren gemäß einem der Ansprüche 7-9, wobei die Verifizierung der Authentizität einer Vielzahl von gemessenen ESR-Spektren in einem gemeinsamen Nachweisschritt durchgeführt wird.

11. Verfahren gemäß einem der Ansprüche 7-10, wobei der Verfahrensschritt (d) mit einem mobilen Endgerät, vorzugsweise einem Smartphone ausgeführt wird, auf welchem ein Computerprogramm ausgeführt wird, das unter Verwendung der Schaltkreiskomponenten des mobilen Endgerätes ein ESR-Spektrum des Produkts misst, wobei vorzugsweise zur Messung des ESR-Spektrums des Produktes ein zusätzlicher Permanentmagnet oder dazu geeignete Antennen oder externe zuschaltbare Schaltkreiskomponenten verwendet werden.

12. Verfahren zur Verifizierung der Authentizität eines Produktes aufweisend eine paramagnetische Phasen enthaltende Identifizierungs-Stoffzumischung, die ein ESR-Fingerprint-Spektrum aufweist, das eine eindeutige Identifizierung des Produktes erlaubt, aufweisend die Verfahrensschritte:

- Aufnahme eines ESR-Spektrums des Produktes mit einem mobilen Endgerät, vorzugsweise einem Smartphone, auf welchem ein Computerprogramm ausgeführt wird, das unter Verwendung der Schaltkreiskomponenten des mobilen Endgerätes ein ESR-Spektrum des Produkts misst, und
- Vergleich des aufgenommenen ESR-Spektrums des Produktes mit gespeicherten ESR-Fingerprint-Spektren, wobei zur Messung des ESR-Spektrums des Produktes vorzugsweise ein zusätzlicher Permanentmagnet verwendet wird.

13. Vorrichtung zur Authentifizierung eines Produktes aufweisend eine paramagnetische Phasen enthaltende Identifizierungs-Stoffzumischung, die ein ESR-Fingerprint-Spektrum aufweist, das eine eindeutige Identifizierung des Produktes erlaubt, wobei die Vorrichtung aufweist:

- eine Spektrometereinheit eingerichtet zur Messung eines ESR-Spektrums,
- eine Kommunikationseinheit eingerichtet zum Zugriff auf eine Datenbank, welche digitale Repräsentationen von ESR-Fingerprint-Spektren speichert, und
- eine Datenverarbeitungseinheit eingerichtet zur Erstellung einer digitalen Repräsentation des gemessenen ESR-Spektrums des zu authentifizierenden Produktes und zum Vergleich mit den in der Datenbank gespeicherten digitale Repräsentationen von ESR-Fingerprint-Spektren, wobei die Vorrichtung insbesondere als mobiles oder stationäres Endgerät, vorzugsweise als Smartphone, ausgebildet ist, auf welchem ein Computerprogramm ausführbar ist, das unter Verwendung der Schaltkreiskomponenten des mobilen Endgerätes ein ESR-Spektrum des Produkts misst.

14. Verwendung eines mobilen Endgerätes, vorzugsweise eines Smartphones, zur Authentifizierung eines Produktes aufweisend eine paramagnetische Phasen enthaltende Identifizierungs-Stoffzumischung, die ein ESR-Fingerprint-Spektrum aufweist, auf welchem mobilen Endgerät ein Computerprogramm ausgeführt wird, das unter Verwendung der Schaltkreiskomponenten des mobilen Endgerätes ein ESR-Spektrum des Produkts misst.

15. Computerprogrammprodukt, das auf einem Endgerät, vorzugsweise einem Smartphone, ausführbar ist, welches unter Verwendung der Schaltkreiskomponenten des Endgerätes ein ESR-Spektrum einer paramagnetische Phasen enthaltende Probe misst.

Fig. 1

S1: Aufbringen eine paramagnetische Phasen enthaltenden Identifizierungs-Stoffzumischung

S2: Aufnahme eines ESR-Fingerprint-Spektrums des Produktes

S3: Erzeugung und Speicherung einer digitalen Repräsentation des ESR-Fingerprint-Spektrums

S4: Messung des ESR-Spektrums eines zu authentifizierenden Produktes

S5: Erzeugung einer digitalen Repräsentation des gemessenen ESR-Spektrums

S6: Verifizierung des Produktes durch Vergleich der digitalen Repräsentation des gemessenen ESR-Spektrums des zu authentifizierenden Produktes mit digitalen Repräsentationen gespeicherter ESR-Fingerprint-Spektren

Fig. 2a

Fig. 2a: ESR-Spektren an Pulvergemischen aus UB und MAG in verschiedenen Gewichtsverhältnissen, aufgenommen bei Raumtemperatur

Fig. 2b

Fig. 2b: Zweite Ableitungen der ESR-Spektren aus Fig. 2a nach $H_{appl}$

Fig. 3

Fig. 4

Fig. 5

Fig. 5: ESR-Spektren dünner Filme enthaltend $Fe_3O_4$, Ultramarin Blau, MAG und UB geschichtet

Fig. 6

Fig. 6: ESR-Spektren: a) Ultramarin Blau (UB), b) phen(CuCl₂), c) UB und phen(CuCl₂) im
Gewichtsverhältnis 1:1 gemischt, a) + b) direkte Summe aus a) und b)

Fig. 7

Fig. 7: ESR-Spektren von Magnetit bei verschiedenen Temperaturen

Fig. 8

Fig. 8: ESR-Spektren von Ultramarin bei 100 K bzw. Raumtemperatur

Fig. 9

Fig. 9: ESR-Spektren von Ultramarin und Magnetit als Tablette (a) und suspendierter Tablette (b)

Fig. 10

Fig. 10: ESR-Spektren (normiert auf die gleiche Probenmasse von 50 mg) verschiedener Extrudatproben gemessen bei 20°C. Extrudat 2 zeigt ein schwaches Signal bei etwa 3400 G, das auf $Cu^{2+}$-Arten zurückzuführen sein kann, obwohl keine typische Hyperfeinstruktur von $Cu^{2+}$ sichtbar ist. Der Ursprung der schwachen Signale bei rund 3600 G in den Extrudaten 1 und 2 ist nicht eindeutig. Möglicherweise liegt es an einem paramagnetischen Defekt. Die Extrudate 3 und 4 zeigen ein starkes ESR-Signal von Ultramarin Blau.

Fig. 11

Fig. 11: ESR-Spektren verschiedener Pastenproben (je 50 mg), gemessen bei 20°C, zeigen die ESR-Signale von Ultramarin Blau (Paste 5A und 5B) und Cu²⁺ (Paste 5C)

Fig. 12

Fig. 12: ESR-Spektren verschiedener Pastenproben (je 50 mg) gemischt mit 0,02 mg 2,2,6,6,6-Tetramethyl-1-piperidinyloxyl (TEMPO-Radikal) gemessen bei 20°C. A) TEMPO-Signal; B) Ultramarin Blau-Signal (Paste 5B und 5C; (rote bzw. blaue Linien) und Cu²⁺-Signal (Paste 5C). Die Proben wurden durch Mischen der einzelnen Paste mit der TEMPO-Lösung in Aceton hergestellt; die ESR-Spektren wurden nach der Verdampfung von Aceton (100 µL) gemessen

Fig. 13

Fig. 13: ESR-Spektren verschiedener Pastenproben (je 50 mg) gemischt mit 20 mg $TiO_2/SiO_2$ gemessen bei 20°C. Die Proben wurden durch Mischen der einzelnen Pasten mit festem $TiO_2/SiO_2$ hergestellt

## Fig. 14

Fig. 14: ESR-Spektren verschiedener Pastenproben (je 40 mg) gemischt mit 10 mg MgO mit einer Mn(II)-Verunreinigung (Mn in MgO < 1%) gemessen bei 20°C. A) ESR-Signal des isolierten $Mn^{2+}$-Ions in der MgO-Matrix; B) ESR-Spektren von (A), jedoch mit unterschiedlichem Bereich von X- und Y-Achsen, um das schwach überlagerte EPR-Signal von Ultramarin zu zeigen (zum Vergleich wurden die ESR-Spektren von Paste C und B in diese Abbildung aufgenommen)

Fig. 15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202007017753 U1
- DE 10030629 A1
- US 20060054825 A1
- DE 4445004 A1
- DE 102008060675 A1
- US 20180335427 A1

**In der Beschreibung aufgeführte Nicht-Patentliteratur**

- **ANGELIKA BRÜCKNER.** *Chem. Ing. Tech.,* 2014, vol. 86 (11), 1871-1882
- **DORFMAN.** *J. Exp. Theor. Phys.,* 1965, vol. 48, 715
- **B. HEINRICH J.F. COCHRAN.** *Advances in Physics,* 1993, vol. 42, 523
- **V.K. SHARMA ; F. WALDNER.** *J. Appl. Phys.,* 1977, vol. 48, 4298
- **G. VALLEJO-FERNANDEZ et al.** *J. Phys. D: Appl. Phys.,* 2013, vol. 46, 312001
- **J. SALADO et al.** *J. Non-Crystalline Solids,* 2008, vol. 354, 5207
- **R. BERGER.** *J. Magn. Magn. Mater.,* 2001, vol. 234, 535
- **E. KIM.** *Electron Spin Resonance Shift and Linewidth Broadening of Nitrogen-Vacancy Centers in Diamond as a Function of Electron Irradiation Dose*
- **W. TANG ; W. WANG.** *Meas. Sci. Technol.,* 2011, vol. 22, 1-8